# EUROPEAN PATENT SPECIFICATION

(11) **EP 0 643 685 B1**
(45) Date of publication and mention of the grant of the patent: **22.01.1997**
(21) Application number: 93914336.8
(22) Date of filing: 03.06.1993
(51) Int. Cl.: C07C 213/04, C07C 215/24, C07D 295/08, C07D 521/00

(54) **PALLADIUM CATALYZED ADDITION OF AMINES TO 3,4-EPOXY-1-BUTENE**
PALLADIUM KATALYSIERTE ADDITION VON AMINEN ZU 3-4-EPOXY-BUTEN
ADDITION D'AMINES, CATALYSEE PAR PALLADIUM, A 3,4-EPOXY-1-BUTENE

(30) Priority: 05.06.1992 US 893725; 04.09.1992 US 940983
(43) Date of publication of application: 22.03.1995
(73) Proprietor: EASTMAN CHEMICAL COMPANY, Kingsport, TN 37660 (US)
(72) Inventor: GODLESKI, Stephen, A., Fairport, NY 14450 (US); HUNG, Yann, Rochester, NY 14609 (US)
(74) Representative: Wibbelmann, Jobst, Dr., Dipl.-Chem.
(86) International application number: US9305262
(87) International publication number: WO9325516

(56) References cited:
- US-A- 2 497 553
- US-A- 2 533 085
- JOURNAL OF THE AMERICAN CHEMICAL SOCIETY, vol. 103, no. 19, 23 September 1981, Gaston, PA, US, pages 5969-72; B. M. TROST et al, "Neutral Alkylations via Palladium(0) Catalysis"
- JOURNAL OF THE AMERICAN CHEMICAL SOCIETY, vol. 103, no. 19, 23 September 1981, Gaston, PA, US, pages 6053-4; B. M. TROST et al, "A Synthesis of Substituted Pyrrolidinones via a Palladium (2+) Catalysed Cyclization. An Unusual Approach to a Carbapenem"
- TETRAHEDRON LETTERS, vol. 22, no. 27, 1981, Oxford, GB, pages 2575-8; J. TSUJI et al, "Regioselective 1,4-Addition of Nucleophiles to 1,3-Diene Monoepoxides Catalysed by Palladium Complex"

## Description

A process is disclosed for reacting 3,4-epoxy-1-butene with an amine to produce a 4-amino-substituted-2-buten-1-ol.

A variety of 4-aminobutenols are known to have use themselves or as intermediates for the preparation of other chemical substances. For example, 4-dimethylamino-2-buten-1-ol could be used in the preparation of 4-dimethylaminobutyraldehyde diethyl acetal, an intermediate for a pharmaceutical compound, as disclosed in German Patent No. 3,700,408.

3,4-epoxy-1-butene (butadiene monoepoxide) is one potential substrate for the production of 4-aminobutenols. However, the reaction of nitrogen nucleophiles (e.g., amines) with 3,4-epoxy-1-butene typically yields a mixture of the 1,2 addition product (1,2 adduct) and the 2,1 addition product (2,1 adduct), and little or none of the 1,4 addition product (1,4 adduct). The reaction is illustrated below: 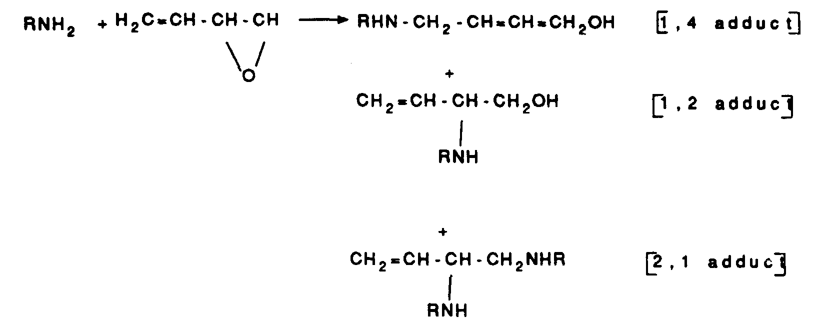 See, e.g., M. G. Ettlinger, "Synthesis of the Natural Antithyroid Factor 1-5-Vinyl-2-Thiooxazolidone", 72 J. Amer. Chem. Soc. 4792-4795 (1950); United States Patent No. 2,533,085 to Blicke; W. B. Wheatley, et al., "o-Benzylphenyl Derivatives. IV. beta-Chloroethylamines", 72 J. Amer. Chem. Soc. 1655-1659 (1950); M. P. Crozet and W. Kassar, "Synthesis and Radical Cyclization of Benzazepinoethane Thiols", 99 Compte Rendu Acad. Sci. Paris 99-101 (1985); United States Patent No. 2,497,553 to Long, Jr.; A. A. Petrov and V. M. Albitskaya, "The Reaction of Divinyloxide with Amines", 26 Zhur. Obshchei Khim. 2125-2127 (1956); and F. F. Blicke and J. H. Biel, "Aminolysis Products of 1-Chloro-2-Hydroxy-3-butene, 1-Hydroxy-2-Chloro-3-butene and 1,2-Epoxy-3-butene", 79 J. Amer. Chem. Soc. 5508-12 (1957).

The regioselective addition of nitrogen nucleophiles to substrates to form 4-aminobutenols (the 1,4 adduct product) is extremely unpredictable in both result and yield. For example, in Tsuji, et al., "Regioselective 1,4-Addition of Nucleophiles to 1,3-Diene Monoepoxides Catalyzed by Palladium Complex", 22 Tetrahedron Letters 2575-78 (1981), the formation of the 1,4 adduct from the palladium catalyzed reaction of 3,4-epoxy-1-dodecene and pyrrolidine is disclosed. Also, Trost, et al., "A Synthesis of Substituted Pyrrolidines via a Palladium(2+) Catalyzed Cyclization. An Unusual Approach to a Carbapenem", 108 J. Amer. Chem. Soc. 6053-54 (1986) discloses the formation of the 1,4 adduct from the addition of a nitrogen nucleophile to 3,4-epoxy-1-butene. On the other hand, Tsuda, et al., "palladium(0)-Catalyzed Reaction of Methyl-g, d-epoxy-sorbate with Nitrogen Nucleophiles", 54 Journal of Organic Chemistry 977-979 (1989) discloses that the palladium-catalyzed reaction of methyl-g, d-epoxysorbate with nitrogen nucleophiles is not regioselective for the 1,4 adduct.

It is widely recognized that the control of regiochemistry in the addition of nucleophiles to an intermediate unsymmetrical allyl-palladium substrate is an extremely complex problem. Many factors enter into the determination of which terminus of the electrophilic allyl ligand the nucleophile will preferentially attack. These factors include the steric nature of the nucleophile (see B. M. Trost, et al., "Allylic Alkylation: Nucleophilic Attack on pi-Allyl Palladium Complexes", 100 J. Amer. Chem. Soc. 3416 (1978) and the allyl substrate (see E. Keinen, et al. "Regioselectivity in Organo-transition-metal Chemistry. A Remarkable Steric Effect in pi-Allyl Palladium Chemistry", J. Chem. Soc., Chem. Commun., 648 (1984)); the electronic nature of the allyl substrate on the palladium (see J. Tsuji, et al., "Palladium-Catalyzed Regioselective Reactions of a-Acetoxy-b, g,- Unsaturated Nitriles and g-Acetoxy-a, b-Unsaturated Ester with Nucleophiles," 22 Tetrahedron Letters 2573 (1981)); the electronic nature of the other ligands on the palladium (see B. Akermark, et al., "Reactivity and syn-anti Isomerization of η-3-geranyl and η-3-neryl Palladium Complexes. Evidence for Electronic Control of the Regiochemistry of Nucleophilic Addition", 4 Organometallics 1275 (1985)); and the mechanism of the addition of the nucleophile (see Godleski, et al., "(pi-Allyl)Palladium Complexes of Norcamphene. Structure and Reactivity", 3 Organometallics 21 (1984)).

B. Trost and E. Keinan, "Steric Steering with Supported Palladium Catalysts", 100 J. Amer. Chem. Soc. at 7779 (1978) ("Keinan") discloses that using a polymer-bound catalyst may have some effect on the stereoselectivity of primary amine addition to cis-3-acetoxy-5-carbomethoxy-1-cyclohexene. However, the relationship of stereochemistry and regiochemistry is very complex and unpredictable. See, e.g., B. M. Trost, 4 Comprehensive Organic Synthesis 585-662 (1991) ("Trost") (discussing nucleophiles with allyl metal complexes). In addition, regiochemical control of the amination of allyl-palladium complexes is known to be particularly unpredictable. See, e.g., B. Akermark, et al., "Amination of pi-Allylpalladium Chloride Complexes. A Mechanistic Study", 103 J. Amer. Chem. Soc. 3037-3040 (1981).

Keinan also discloses that the use of a polymer-bound catalyst may have some effect on the regioselectivity of the addition of carbon nucleophiles to sorbyl acetate. However, amine nucleophiles often behave quite differently than carbon nucleophiles, as discussed in Trost. This fact, as well as the chemical disparity between sorbyl acetate and 3,4 epoxybutene, leave in doubt what effect a polymer-bound catalyst might have on the addition of amines to 3,4-epoxybutene.

Therefore, in light of the usefulness of 4-amino-substituted-2-buten-1-ols derived from 3,4-epoxy-1-butene and the inherent unpredictability of the result (relative to both the product and the yield) of attempted regioselective amine additions to 3,4-epoxy-1-butene, there continues to be a need for predictable, efficient synthetic processes for deriving 4-amino-substituted-2-buten-1-ols from 3,4-epoxy-1-butene. Even small relative increases in the yield of the 1,4 adduct from amine addition to 3,4-epoxy-1-butene can translate into large cost savings when the increase is extrapolated out to production scale.

A process for producing improved yields of 4-amino-substituted-2-buten-1-ols is disclosed. More particularly, the palladium-catalyzed 1,4 addition of amines to 3,4-epoxy-1-butene is described. The present process includes the steps of reacting a quantity of 3,4-epoxy-1-butene with a quantity of an amine, primary or not, in a reaction medium and in the presence of a catalyst under conditions effective to form 4-amino-substituted-2-buten-1-ol. The catalyst utilized in the present process is a complex of palladium and phosphine ligands.

The present invention provides a method for forming 4-amino-substituted-2-buten-1-ols with a greater degree of regioselectivity and in yields that, especially at production levels, are significantly improved. In addition, the present method allows recovery of the catalyst. These factors combine to provide an improved, more cost-effective process for producing 4-amino-substituted-2-buten-1-ols.

The process of the present invention relates to the formation of 4-amino-substituted-2-buten-1-ols from 3,4-epoxy-1-butene. The process includes the steps of providing a quantity of 3,4-epoxy-1-butene and a quantity of an amine, primary or not. The amine and the 3,4-epoxy-1-butene are reacted in a reaction medium in the presence of a catalyst. The catalyst is a complex of palladium ("Pd") and phosphine ligands.

The 3,4-epoxy-1-butene used in the present method can be synthesized by any conventional process. Preferred processes for the preparation of 3,4-epoxy-1-butene are disclosed in United States Patent Nos. 4,897,498 to Monnier et al. and 4,950,773 to Monnier et al.

The amines which will react with 3,4-epoxy-1-butene to form 4-amino-2-buten-1-ol include any lower primary amine, secondary amine, and heterocyclic amine. Primary amines useful in the present method have the general formula R₁-NH₂, wherein R₁ is a substituted or unsubstituted alkyl group having from one to twenty carbons and also hydrogen. Examples of useful primary amines include alkylamines (e.g., methylamine, ethylamine, butylamine, hexylamine, octylamine, decylamine, and the like), alkenylamines (e.g., allylamine, 2-hexenylamine, 4-decenylamine, and the like), alkanolamines (e.g., ethanolamine, octanolamine, and the like), arylamines (e.g., aniline and the like), and cycloalkylamines (e.g., cyclohexylamine, cyclopentylamine, and the like),

Secondary amines useful in the present method have the general formula 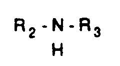 wherein R₂ and R₃ are each a substituted or unsubstituted alkyl group having from one to twenty carbons or a substituted or unsubstituted aryl group having from six to ten carbons, and R₂ and R₃ can be the same or different. Examples of useful secondary amines include dialkylamines (e.g., ethylmethylamines, diethylamines, butylpropylamines, di-n-propylamines, isobutylpropylamines, dihexylamines, octylhexylamines, and the like), dialkenylamines (e.g., allylhexenylamines, diallylamines, and the like), dialkanolamines (e.g., diethanolamine, and the like), N,N'-dialkylalkylenediamines (e.g., N,N'-di-methylethylenediamine, and the like), and dicycloalkylamines (e.g., dicyclohexylamine, cyclopentylhexylamine, and the like).

Heterocyclic amines are also useful secondary amines. Preferably, the heterocyclic amines are in the form of six-membered rings. Exemplary heterocyclic amines include morpholine, piperidine, pyrrolidine, N-methylpiperazine, hexamethyleneimine, and thiomorpholine. The heterocyclic amine may contain additional hetero-atoms including nitrogen, oxygen or sulfur.

The catalyst utilized in the present method is a complex of palladium and phosphine ligands. The catalyst selection should be based on the nature of the reaction medium and amine used.

A catalyst used in the present invention for all types of amines is a polymer-bound complex of palladium with up to four phosphine ligands. Palladium is in its zero oxidation state. Palladium in its zero oxidation state can be used directly or palladium(II) can be used, provided that it generates palladium(0) in situ. Each phosphine ligand provides a tri-substituted phosphorous atom (P) bonded directly to the palladium atom (Pd). At least one phosphine ligand also has one substituent which is a polymer. It is possible for more than one polymer bound phosphine to be ligated to the metal. A palladium atom in a zero oxidation state in a complex is assigned ten valence electrons. It requires eight additional electrons in order to attain an inert gas configuration. This configuration can be achieved by the donation of two electrons by each of four phosphine ligands. However, palladium(0) complexes can exist with only three phosphine ligands (16 electrons) or, to a lesser extent, with two phosphine ligands (14 electrons) and be stable and catalytically active. The catalysts useful in the present method can have the following general formula: 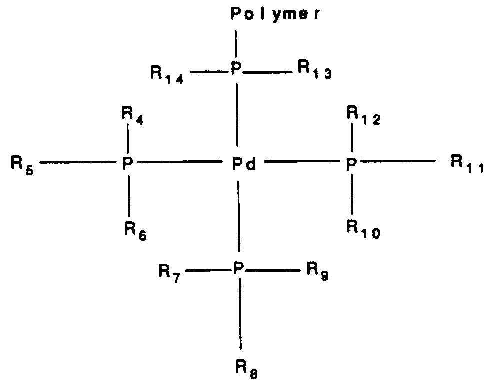 wherein R₄, R₅, R₆, R₇, R₈, R₉ R₁₀, R₁₁ and R₁₂ (collectively "R₄-R₁₂") can be the same or different and are substituted or unsubstituted hydrocarbons, preferably with no more than one of R₄-R₁₂ containing more than seven carbon atoms. In an especially preferred embodiment, R₄-R₁₂ are all phenyl moieties. R₁₃ and R₁₄ can be the same or different and are substituted or unsubstituted hydrocarbons. Preferably R₁₃ and R₁₄ are phenyl, tolyl, or a heterocyclic such as furyl.

In addition to the general formula above, two phosphines can be bound at the same polymer site to form a bidentate ligand according to formula II: 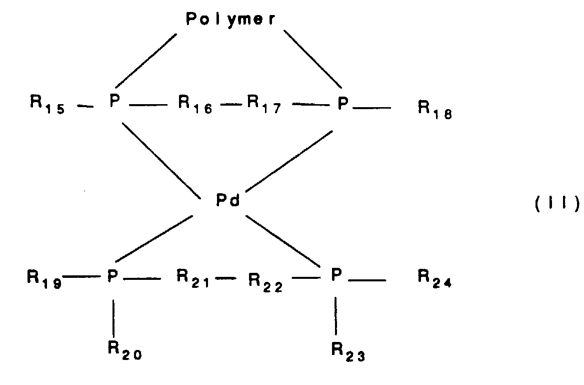 wherein R₁₉, R₂₀, R₂₁, R₂₂, R₂₃ and R₂₄ (collectively "R₁₉-R₂₄") can be the same or different and are substituted or unsubstituted hydrocarbons, preferably with no more than one of R₁₉-R₂₄ containing more than seven carbon atoms. In an especially preferred embodiment, R₁₉-R₂₄ are phenyl moieties. R₁₅, R₁₆, R₁₇, and R₁₈ can be the same or different and are substituted or unsubstituted hydrocarbons. Preferably, R₁₅, R₁₆, R₁₇ and R₁₈ are phenyl, tolyl, or a heterocyclic such as furyl.

Exemplary polymers to which the palladium catalyst can be bound include polystyrene and, most preferably, diphenylphosphine derivatized polystyrene beads crosslinked with divinylbenzene as disclosed in B. M. Trost and E. Keinen, "Steric Steering with Supported Palladium Catalysts", 100 J. Am. Chem. Soc. 7779 (1978) ("Trost") and C. U. Pittman and Q. Ng, "Use of Polymer Matrices to Activate Palladium(0) Catalysts and Reduce Catalyst Agglomeration", 153 J. Organomett. Chem. 85 (1978), the disclosures of which are hereby incorporated by reference. Preferably, polystyrene beads used as a support for the palladium catalyst in the present method should be about 15-300 mm in diameter, most preferably about 35-250 mm. It is also advantageous, although not required, to crosslink the polystyrene beads with divinylbenzene. Divinylbenzene can be added in a concentration of about 1-10 weight percent ("wt%"), preferably about 8-10 wt%. Preferably, the ratio of phosphine to palladium on the polystyrene bead is in the range of from about 1:1 to about 20:1, as determined by Inductively Coupled Plasma Atomic Emission Spectrometry. Most preferably, the phosphine to palladium ratio on the bead can be from about 3:1 to 6:1. The polystyrene beads can be made by suspension polymerization as described in P. Hodge and D. C. Sherrington, Polymer-Supported Reactions in Organic Synthesis, Appendix, 469-75 (1980). Suitable polystyrene beads are also commercially available as Cat. No. 4020, 200-400 mesh, from Polysciences, Inc., Warrington, PA.

The following references disclose exemplary processes useful in forming diphenylphosphine derivatized polystyrene beads crosslinked with divinylbenzene: "Supported Transition Metal Complexes as Catalysts", 15 Adv. Organomet. Chem. 189 (1977) (procedure for washing the beads); "[Poly(styryl)bipryidine] palladium(0)-Catalyzed Isomerization of Quadricyclene", 43 J. Org. Chem. 2958 (1978) (procedure for bromination of the beads); "Comparative Study of Some Selected Reactions of Homogeneous and Polymer-Supported Lithium Diorganocuprates", 44 J. Org. Chem. 2705 (1979) (procedure for phosphorylation of the beads); 153 J. Organomet. Chem. 85 (1978), 100 J. Am. Chem. Soc. 7779 (1978) (procedure for loading palladium on the beads).

Palladium(0) species can also be supported on: alumina, silica gel according to the method described in Trost; polyvinyl chloride, polybutadiene, or polyvinyl alcohol as described in K. G. Allum et al., "Supported Transition Metal Complexes I. Organic Polymers as the Support", 87 J. Organomet. Chem. 189 (1975); a copolymer of styrene, methacrylate, and ethylene 1,2-dimethacrylate (a cross-linking monomer) as described in R. Deschenaux, J. Stille, "Transition-Metal-Catalyzed Asymmetric Organic Synthesis via Polymer-Attached Optically Active Phosphine Ligands. 13. Asymmetric Hydrogenation with Polymer Catalysts Containing Primary and Chiral Secondary Pendant Alcohols", 50 J. Org. Chem. 2299 (1985), as well as other copolymers; or on cellulose as described in "Effect of Acid on the Radiation-Induced Copolymerization of Monomers to Cellulose", 11 J. Polym. Sci., Polym. Lett. Ed. 711 (1973). An example of a useful commercially available palladium complex supported on a polymer substrate is polymer-supported tetrakistriphenylphosphinepalladium(0), Catalog No. 24815-0, available from Aldrich Chemical Co., Inc.

The polymer bound catalyst of the present invention not only increases the yield of the 1,4 adduct, but has the additional advantage of protecting the catalyst against loss in use by facilitating extraction of the catalyst from the reaction system.

In contrast to the catalyst described above, it is preferable when the amine is primary, the catalyst should be substantially soluble in the reaction medium to maximize the catalysts' activity. For example, if water is the reaction medium, a water soluble palladium catalyst is preferred. On the other hand, if the reaction medium is less polar than water (e.g., an organic solvent) the catalyst is preferably substantially soluble in that medium. If the palladium catalyst has phosphine ligands that are in their liquid state at the reaction temperature, the relative solubility of the catalyst is not critical.

The primary amine catalyst can either be added to the reaction medium in a catalytically active form or in the form of precursors that are added separately to the reaction medium and become catalytically active in situ. If added as precursors, the catalyst precursors include a palladium precursor and phosphine ligands. The palladium precursor and the phosphine ligands are mixed in a small amount of a solvent (which both are soluble in) to form the catalyst in situ. This catalyst solution is then added to the reaction medium. When the catalyst is in the form of catalyst precursors, the phosphine ligands utilized in conjunction with the palladium precursor have the general formula PR₁R₂R₃, wherein P is phosphorus and R₁, R₂, and R₃ can be the same or different and each are an alkyl, cycloalkyl, alkoxide, aryl, heterocyclic, functionalized alkyl, functionalized aryl, or ether group.

For the purposes of the present invention, useful palladium catalysts for primary amines have been grouped into eight general catgories (i.e., Categories I-VIII). Within the description of each category, Q is a cation such as Na⁺, K⁺, Li⁺, (CH₃)₄N⁺, NH₄ ⁺, and the like. X is an anion such as Cl⁻, Br⁻, NO₃ ⁻, NO₂ ⁻, N₃ ⁻, BF₄ ⁻, OH⁻, SCN⁻, b-diketonate, carboxylate, and the like. Ph represents a phenyl group. P is phosphorus and Pd is palladium. a is a number in the range from 1 to 3; b is a number in the range from 1 to 4; c and f are 1 or 2; d is a number in the range from 2 to 4; and e is either 12 or 16. R₁, R₂, and R₃ are defined above.

Category I palladium catalysts have the formula Pd(Pr₁R₂R₃)₂X₂. Useful Category I catalysts include, for example
Pd(PPh₃)₂X₂,
Pd(P(p-tolyl)₃)₂X₂,
Pd(PhP(C₂H₄CN)₂)₂X₂, and
Pd(P(C₂H₄CN)₃)₂X₂.
Especially useful Category I catalysts include
Pd(Ph₂P(m-C₆H₄SO₃Q))₂X₂,
Pd(P(m-C₆H₄SO₃Q)₃)₂X₂,
Pd(Ph₂P(o-CO₂C₆H₄Q))₂X₂,
Pd(P(CH₂(CH₂CH₂O)ₐCH₃)₃)₂X₂,
Pd(PhP(CH₂CH₂CH₂OCH₃)₂)₂X,
Pd(Ph₂PCH₂CH₂N(CH₃)₃X)₂X₂,
Pd(Ph₂PCH₂CH₂CO₂Q)X₂, and the like. The preparation of Category I catalysts is described in D. Hedden et al, "Synthesis of New Hybrid Phosphine Amine and Phosphine Amide Compounds. Preparation of a Series of New Phosphine Amido Chelate Complexes of Palladium (II) and Platinum (II) and Their Reactions with Bases and Bronsted Acids", 24 Inorg. Chem., 4152-4158 (1985) and C. Larpent and H. Patin, "Hydrosoluble Transition-metal Coordination Compounds of Triphenylphosphine m-Trisulfonate". 1 Appl. Organomet. Chem. 529 (1987).

Category II catalysts have the general formula Pd(R₄R₅P(V)_{b}ZR₆R₇)X₂ wherein V is CH₂ or CH as part of unsaturated component; Z is phosphorus or nitrogen; and R₄, R₅, R₆, and R₇ can be the same or different and each are an alkyl, cycloalkyl, alkoxide, aryl, heterocyclic, functionalized alkyl, functionalized aryl, or ether group. Useful Category II catalysts include
Pd[(C₆H₅)₂P(CH₂)P(C₆H₅)₂]X₂,
Pd[(C₆H₅)₂P(CH₂)₂P(C₆H₅)₂]X₂,
Pd[(C₆H₅)₂P(CH₂)₃P(C₆H₅)₂]X₂,
Pd[(C₆H₅)₂P(CH₂)₄P(C₆H₅)₂]X₂,
Pd[(p-tolyl)₂P(CH₂)P(p-tolyl)₂]X₂,
Pd[(p-tolyl)₂P(CH₂)₂P(p-tolyl)₂]X₂,
Pd[(C₆F₅)₂P(CH₂)₂P(C₆F₅)₂]X₂,
Pd[(NCC₂H₄)₂PC₂H₄P(C₂H₄CN)₂]X₂, and the like.
Especially useful Category II catalysts include
Pd[(C₆H₅)₂P(CH₂)₂N(CH₃)₂]X₂,
Pd[(m-SO₃C₆H₄Q)_{c}(C₆H₅)_{2-c}P(CH₂)_{d}P(C₆H₅)_{f} (m-SO₃C₆H₄Q)_{2f}]X₂,
Pd[(m-SO₃C₆H₄Q)_{c}(C₆H₅)_{2-c}PCH(CH₃)CH(CH₃)P(C₆H₅)_{f}-(m-SO₃C₆H₄Q)_{2-f}]X₂,
Pd[(p-(CH₃)₃NC₆H₄X)₂PC₂H₄P(p-(CH₃)₃NC₆H₄X)₂]X₂,
Pd[(p-(CH₃)₃NC₆H₄X)₂PCH(CH₃)CH(CH₃)P(p-(CH₃)₃-NC₆H₄X)₂]X₂, and 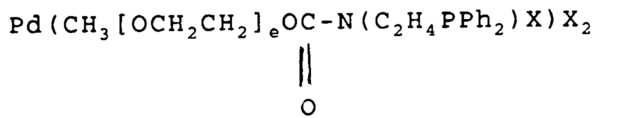
The preparation of Category II catalysts is described in M. J. Hudson, et al, "Ditertiary Arsine and Phosphine Compounds of Nickel (II), Palladium (II), and Platinum (II)", J. Chem. Soc., A, 40 (1968).

Category III catalysts have the general formula [(η³-CH(R₈)C(R₉)CH(R₁₀))Pd(PR₁R₂R₃)₂]X, wherein R₈, R₉, and R₁₀ are the same or different and each are hydrogen, alkyl, cycloclkyl, aryl, heterocyclic, halogen, ether, sulfide, carbonyl or are linked together to form a ring. Useful Category III catalysts include
[(η³-C₃H₅)Pd(PPh₃)₂](X),
[(η³-C₃H₅)Pd(P(p-tolyl)₃)₂](X),
[(η³-C₃H₅)Pd(P(C₄H₃0)₃)₂](X), and the like.

Especially useful Category III palladium catalysts include
[(η³-C₃H₅)Pd(P(m-C₆H₄SO₃Q)₃)₂](X),
[(η³-C₃H₅)Pd(Ph₂P(m-C₆H₄SO₃Q))₂](X),
[(η³-C₃H₅)Pd(Ph₂P(m-C₆H₄CO₂Q))₂]X,
[(η³-C₃H₅)Pd(P(CH₂(CH₂CH₂O)ₐCH₃)₃)₂]X,
[(η³-C₃H₅)Pd(PhP(CH₂CH₂OCH₃)₂)₂]X,
[(η³-C₃H₅)Pd(Ph₂PCH₂CH₂N(CH₃)₃X)₂]X,
[(η³-C₃H₅)Pd(P(O-iso-C₃H₇)₃)₂]X, and the like.

Category IV catalysts have the general formula [(η³-CH(R₈)C(R₉)CH(R₁₀))Pd(R₄R₅P(V)_{b}ZR₆R₇]X, wherein R₄, R₅, R₆, R₇, V, and Z have the same meanings as described above for the Category II catalysts; and R₈, R₉, and R₁₀ have the same meanings as described above for the Category III catalysts. Useful Category IV catalysts include
[(η³-C₃H₅)Pd(C₆H₅)₂P(CH₂)P(C₆H₅)₂](X),
[(η³-C₃H₅)Pd(C₆H₅)₂P(CH₂)₂P(C₆H₅)₂](X),
[(η³-C₃H₅)Pd(C₆H₅)₂P(CH₂)₃P(C₆H₅)₂](X), and the like.
Especially useful Category IV catalysts include
[(η³-C₃H₅)Pd[(m-SO₃C₆H₄Q)_{c}(C₆H₅)_{2-c}P(CH₂)_{d}P(C₆H₅)_{f}-(m-SO₃C₆H₄Q))_{2-f}]X,
[(η³-C₃H₅)Pd(p-(CH₃)₃NC₆H₄X)₂PC₂H₄P(p-(CH₃)₃NC₆H₄X)₂]X,
[(η³-C₃H₅)Pd(p-(CH₃)₃NC₆H₄X)₂PCH(CH₃)CH(CH₃)P-(p-(CH₃)₃NC₆H₄X)₂]X.
The preparation of Category III and IV catalysts is described in B. Akermark, et al, "Ligand Effects and Nucleophilic Addition to (η-³-allyl)Palladium Complexes. A Carbon-13 Nuclear Magnetic Resonance Study", 6 Organometallics 620 (1987).

Category V catalysts have the general formula Pd(R₄R₅P(V)_{b}ZR₆R₇)₂, wherein R₄, R₅, R₆, R₇, V, and Z are as described above for Category II catalysts. Useful Category V catalysts include Pd[(C₆H₅)₂P(CH₂)₂P-(C₆H₅)₂]₂, and the like. Especially useful Category V catalysts include
Pd[(C₆H₅)₂P(CH₂)₂N(CH₃)₂]₂,
Pd[(p-(CH₃)₃NC₆H₄X)₂PCH(CH₃)CH(CH₃)P-(p-(CH₃)₃NC₆H₄X)₂]₂,
Pd[(p-(CH₃)₃NC₆H₄X)₂PC₂H₄P(p-(CH₃)₃NC₆H₄X)₂]₂, and
Pd[(m-SO₃C₆H₄Q)_{c}(C₆H₅)_{2-c}P(CH₂)_{d}P(C₆H₅)_{f}-(m-SO₃C₆H₄Q)_{2-f}]₂.

Category VI catalysts have the general formula Pd(RP₁R₂R₃)₄. Useful Category VI catalysts include
Pd(P(m-C₆H₄SO₃Q)₃)₄,
Pd[Ph₂P(m-SO₃C₆H₄Q)]₃,
Pd[Ph₂P(m-SO₃C₆H₄Q)]₄,
Pd[P(CH₂(CH₂CH₂O)ₐCH₃)₃]₄,
Pd[P(CH(CH₃)(CH₂CH₂O)₃CH₃)₃]₄,
Pd[PhP(CH₂CH₂CH₂OCH₃)₂]₄, and
Pd[Ph₂PCH₂CH₂N(CH₃)₃X]₄.
The preparation of Category V and VI catalysts is described in A. L. Casalnuovo and J. C. Calabrese, "Palladium-Catalyzed Aklylation in Aqueous Media", 112 J. Amer. Chem. Soc. 4324 (1990).

Category VII catalysts have the general formula [Pd(D)(PR₁R₂R₃)₂]X₂, wherein D is a cyclic diene. Useful Category VII catalysts include [(C₈H₁₂)Pd(PPh₃)₂]X₂, and [(C₇H₈)Pd(PPh₃)₂]X₂ (wherein the cyclic diene is bicyclo[2,2,1]hepta-2,5-diene) and cis, cis-1,5-cyclo-octadiene.
Especially useful Category VII catalysts include
[(C₈H₁₂)Pd(P(m-C₆H₄SO₃Q)₃)₂]X₂,
[(C₈H₁₂)Pd(Ph₂P(m-C₆H₄SO₃Q))₂]X₂,
[(C₇H₈)Pd(P(CH₂(CH₂CH₂O)ₐCH₃)₃)₂]X₂,
[(C₇H₈)Pd(PhP(CH₂CH₂CH₂OCH₃)₂)₂)X₂,
[(C₇H₈)Pd(Ph₂PCH₂CH₂N(CH₃)₃X)₂]X₂,
[(C₇H₈)Pd((C₆H₅)₂P(m-SO₃C₆H₄Q))₂]X₂,
[(C₇H₈)Pd(P(m-SO₃C₆H₄Q)₃)₂]X₂,
[(C₈H₁₂)Pd(P(CH₂(CH₂CH₂O)ₐCH₃)₃)₂]X₂, and
[(C₈H₁₂)Pd(PhP(CH₂CH₂CH₂OCH₃)₂)₂]X₂.

Category VIII catalysts have the general formula [Pd(D)(R₄R₅(V)_{b}ZR₆R₇)]X₂, wherein D is as described above for Category VII, and R₄, R₅, R₆, R₇, V, and Z are as described above for Category II. Useful Category VIII catalysts include
[(C₇H₈)Pd(C₆H₅)₂P(CH₂)_{b}P(C₆H₅)₂]X₂, and
[(C₈H₁₂)Pd(C₆H₅)₂P(CH₂)_{b}P(C₆H₅)₂]X₂.
Especially useful Category VIII catalysts include
[(C₇H₈)Pd((p-(CH₃)₃NC₆H₄X)₂PCH(CH₃)CH(CH₃)P (p-(CH₃)₃NC₆H₄X)₂]X₂,
[(C₇H₈)Pd(m-SO₃C₆H₄Q)_{c}(C₆H₅)_{2-c}P(CH₂)_{d}P(C₆H₅)_{f} (m-SO₃C₆H₄Q)_{2-f}]X₂,
[(C₇H₈)Pd(p-(CH₃)₃NC₆H₄X)₂PC₂H₄P(p-(CH₃)₃NC₆H₄X)₂]X₂, 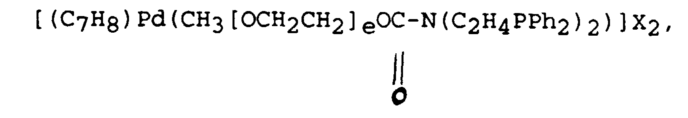
[(C₇H₈)Pd(m-SO₃C₆H₄Q)_{c}(C₆H₅)_{2-c}PCH(CH₃)CH(CH₃)P(C₆H₅) _{f}(m-SO₃C₆H₄Q)_{2-f}]X₂,
[(C₈H₁₂)Pd((p-(CH₃)₃NC₆H₄X)₂PCH(CH₃)CH(CH₃)P (p-(CH₃)₃NC₆H₄X)₂]X₂,
[(C₈H₁₂)Pd(p-(CH₃)₃NC₆H₄X)₂PC₂H₄P(p-(CH₃)₃NC₆H₄X)₂]X₂, 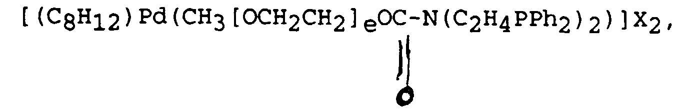
[(C₈H₁₂)Pd(m-SO₃C₆H₄Q)_{c}(C₆H₅)_{2-c}P(CH₂)_{d}P(C₆H₅) _{f}(m-SO₃C₆H₄Q)_{2-f}]X₂,
[(C₈H₁₂)Pd(m-SO₃C₆H₄Q)_{c}(C₆H₅)_{2-c}PCH(CH₃)CH(CH₃)P(C₆H₅) _{f}(m-SO₃C₆H₄Q)_{2-f}]X₂.
The preparation of Category VII and Category VIII catalysts is described in D. A. White, "Cationic Diene Complexes of Palladium (II) and Platinum (II)", v. XIII Inorg. Synthesis. 56 (1972).

A wide variety of common organic liquid solvents can be used as the reaction medium in the inventive method. Useful solvents include tetrahydrofuran, diethyl ether, toluene, dimethylformamide, benzene, dimethylsulfoxide, chloroform, dichloromethane, acetonitrile, dioxane, alcohols, 1,2-dimethoxyethane, and acetone. Tetrahydrofuran is a particularly useful solvent because of its volatility which makes it easier to remove after the reaction. Tetrahydrofuran is also able to swell polymer beads to make them accessible to the reactants as well as ligate (and thereby stabilize) the metal.

The reaction medium used in the present method for primary amines preferably is a liquid having an E_{T}(30) no less than about 32 as measured by a method disclosed in C. Reichardt, Solvent Effects in Organic Chemistry, pp. 242-244 (1979) ("Reichardt"), the disclosure of which is hereby incorporated by reference. E_{T}(30) is defined according to Reichardt as the transition energy for the solvatochromic absorption band with the longest wavelength of the dye 2,6-diphenyl-4-(2,4,6-triphenyl-1-pyridino) phenoxide, as measured at 25°C and 760 torr. The E_{T}(30) value quantifies the polarity of a reaction medium and takes into account both the dielectric constant, dipole moment, and the degree of hydrogen bonding exhibited by the liquid. Generally, the higher the E_{T}(30), the more polar the liquid. It has been discovered in accordance with the present invention that reaction media exhibiting a high degree of hydrogen bonding in combination with a high dielectric constant and/or dipole moment allow for the greatest regioselectivity for the 1,4-adduct of the addition reaction of primary amines to 3,4-epoxy-1-butene.

Reaction media useful in the present method (and their E_{T}(30) include, but are not limited to, toluene (33.9), benzene (34.5), diethylether (34.6), 1,4-dioxane (36.0), tetrahydrofuran (37.4), chloroform (39.1), acetone (42.2), dimethylsulfoxide (45.0), acetonitrile (46.0), nitromethane (46.3), 2-propanol (48.6), ethanol (51.9), 2-methoxyethanol (52.3), N-methylformamide (54.1), methanol (55.5), 1,2-ethanediol (56.3), glycerol (57.0), 2,2,2-trifluoroethanol (59.5), and water (63.1). Preferably, the reaction medium has an E_{T}(30) not less than about 40, most preferably not less than about 45. The best reaction medium is water because of its high dielectric constant and very high degree of hydrogen bonding. Other useful reaction media include liquid methylamine and combinations of liquids such as water combined with an organic solvent. Preferably, the combination includes water. An especially useful mixed reaction medium is an 80:20 solution of ethanol and water, which has an E_{T}(30) of 53.7.

The amount of the amine incorporated into the solvent can vary up to the amine's solubility limit. Preferably, the concentration of amine in the solvent is from about 0.1 to about 6 M, most preferably about 3 to about 6 M. In fact, the proportions of the reactants and the catalyst can be varied widely while obtaining the desired reaction product in high yield. Generally, because palladium is a precious metal, low concentrations of catalyst are preferred. Catalyst concentrations as low as 0.01 mole percent, based on 3,4-epoxy-1-butene, can be used. Preferably, the catalyst concentration is at least about 0.05 mole percent, most preferably at least about 0.1 mole percent, based on 3,4-epoxy-1-butene. The catalyst concentration is normally less than 20 mole percent, preferably less than 10 mole percent, based on 3,4-epoxy-1-butene.

The molar ratio of amine to 3,4-epoxy-1-butene can also vary over wide ranges. Molar ratios from 1:10 to 100:1, preferably 1:6 to 50:1, can be used. To maximize the conversion of 3,4-epoxy-1-butene, a stoichiometric excess of the remaining reactant or reactants should be present. Therefore, a particularly preferred range of amine to 3,4-epoxy-1-butene is in the range from about 3:1 to 40:1.

The conversion of 3,4-epoxy-1-butene can occur in a temperature range from about -6° C to 125° C. Preferably, the reaction is performed at room temperature, although mild heating can be used to accelerate the reaction. The methylamine partial pressure can be up to 5 atmospheres.

The invention is further illustrated by the following examples.

### EXAMPLES

### Example 1 --Reaction of 3,4-epoxy-1-butene and diethylamine (catalyzed with unbound catalyst)

5.0 g (4.3 mmole) of unbound tetrakistriphenylphosphine palladium(0) was dissolved in 20 ml tetrahydrofuran ("THF") under a nitrogen atmosphere. The solution was cooled to 0°C. 10 g (0.14 mole) diethylamine and 10 g (0.14 mole) 3,4-epoxy-1-butene were then added. The reaction mixture was stirred at room temperature for 36 hours. The reaction mixture was concentrated by evaporation at reduced pressure. The residue was distilled at reduced pressure. The reaction resulted in an 80% yield of a complex mixture of products. The product was analyzed by capillary gas chromatography ("GC") (HP 5893A Gas Chromatograph, DB5 column, 0.32 mm I.D., 0.25 micron thick, 30 meter, from J & W Scientific). Gas chromatography analysis revealed an approximately 1:1 mixture of 4-(N,N-diethylamino)-2-buten-1-ol (1,4 adduct) and 3-diethylamino-2-buten-4-ol (1,2 adduct).

### Example 2 -- Reaction of 3,4-epoxy-1-butene and diethylamine (catalyzed with polymer-bound catalyst)

0.4 g of polymer-bound triphenylphosphine palladium(0) from Aldrich Chemical Co., Inc., Milwaukee, Wisconsin, Catalogue No. 24815-0 was suspended in 20 ml of THF, followed by 1.42 ml (14.3 mmol) diethylamine and 1.15 ml (14.3 mmol) 3,4-epoxy-1-butene. The reaction mixture was stirred at room temperature for 36 hours. The catalyst was removed by filtration and the filtrate was concentrated by evaporation at reduced pressure. The residue was distilled at reduced pressure. The reaction resulted in an 80% yield of 4-(N,N-diethylamino)-2-buten-1-ol. None of the isomeric 1,2 adduct was detected. The increased ratio of 4-(N,N-diethylamino)-2-buten-1-ol produced as compared to the approximately 1:1 ratio seen in Example 1 (with an unbound catalyst) has tremendous manufacturing advantages when extrapolated out to production scale synthesis.

### Example 3 - Reaction of 3,4-epoxy-1-butene and piperazine

1.23 g (14.3 mmol) of piperazine was dissolved in 20 ml of THF. 0.8 g of the polymer-bound palladium catalyst of Example 2 and 2.3 ml (28.6 mmol) 3,4-epoxy-1-butene was then added to the reaction mixture. The reaction mixture was stirred for 24 hours at room temperature. The catalyst was removed by filtration and the filtrate was concentrated at reduced pressure, resulting in a 98% yield of N,N'-di-(4-hydroxy-2-butenyl)-piperazine (1,4 adduct). None of the isomeric 1,2 adduct was detected.

### Example 4 --Reaction of 3,4-epoxy-1-butene and ethylaminoethanol

0.4 g of the polymer-bound palladium(0) catalyst of Example 2 was suspended in 20 ml of THF. 1.39 ml (14.3 mmol) of ethylaminoethanol and 1.15 ml (14.3 mmol) of 3,4-epoxy-1-butene was added to the reaction mixture. The reaction mixture was stirred for forty-eight hours at room temperature. The catalyst was removed by filtration and the filtrate was concentrated at reduced pressue to yield 85% of the 1,4 adduct, 4-(2-hydroxyethylamino)-2-buten-1-ol (1,4 adduct) with a 2:1 ratio of the trans type olefin to the cisoid olefin) (E:Z ratio of 2:1) and 15% of the 1,2 adduct, 3-(2-hydroxyethylamino)-1-buten-4-ol.

### Example 5 --Reaction of 3,4-epoxy-1-butene and piperidine

6.07 g (0.071 moles) of piperidine and 5.0 g (0.071 moles) 3,4-epoxy-1-butene were dissolved in 50 ml of THF. 1.0 g of the polymer-bound palladium(0) catalyst of Example 2 was then added to the reaction mixture. The resulting slurry was stirred at room temperature for 18 hours. The catalyst was removed by filtration and the filtrate was concentrated at reduced pressure. The residue was distilled at reduced pressure to yield 49.7% 4-(1-piperadino)-2-buten-1-ol (1,4 adduct) and 9% 3-(l-piperadino)-1-buten-4-ol (1,2 adduct).

### Example 6 -- Reaction of 3,4-epoxy-1-butene and N,N'-diethylphenylenediamine

7.45 g (0.045 mole) of N,N'-diethylphenylenediamine and 6.36 g (0.091 mole) 3,4-epoxy-1-butene were dissolved in 70 ml of THF. The polymer-bound palladium(0) catalyst of Example 2 was then added to the reaction mixture. The resulting slurry was heated at reflux at 65° C for 48 hours. The catalyst was removed by filtration and the filtrate was concentrated at reduced pressure. The residue was distilled at reduced pressure to yield N,N'-[diethyl-di-(4-hydroxy-1-butenyl)]-phenylenediamine (1,4 adduct) and N,N'-[diethyl-di-3-(4-hydroxy-1-butenyl)]-phenylenediamine (1,2 adduct) in a 4:1 ratio.

### Example 7 --Reaction of 3,4-epoxy-1-butene and imidazole

1.95 g (0.0286 moles) of imidazole and 2.0 g (0.0286 moles) of 3,4-epoxy-1-butene were dissolved in 25 ml of THF. 0.83 g of the polymer-bound palladium(0) catalyst of Example 2 was then added to the reaction mixture. The reaction mixture was stirred at room temperature for 24 hours. The catalyst was removed by filtration and the filtrate was concentrated at reduced pressure. The residue was distilled. The reaction yielded about 95% 4-(l-imidazoyl)-2-buten-1-ol (1,4 adduct) and less than 5% 3-(l-imidazoyl)-1-buten-4-ol (1,2 adduct) and less than 5% 3-(1-imidazoyl)010buten-4-ol (1,2 adduct). The combined yield of products was 63%.

### Example 8 --Reaction of 3,4-epoxy-1-butene and, methylamine (catalyzed with unbound palladium catalyst)

0.05 g (0.043 mmole) of unbound tetrakistriphenylphosphine palladium(0) was dissolved in a solution of 2.7 ml methylamine in THF (2.8 M) under an argon atmosphere. 0.06 ml (0.75 mmole) of 3,4-epoxy-1-butene was then added and the reaction mixture wa stirred at 30° C for 20 hours. The products of the reaction were approximately 60% 2,5- and 2,6-divinyldioxane. A low yield (7.4%) of the desired product, 4-methylamino-2-buten-1-ol, was present. Approximately 16% of the 1,2 adduct, 2-methylamino-3-buten-1-ol was formed. Therefore, the regioselectivity of the reaction (the ratio of the 1,4 adduct to the 1,2 adduct) was only about 0.45:1.

### Example 9 --Reaction of 3,4-epoxy-1-butene and methylamine (catalyzed with polymer-bound palladium catalyst)

A palladium catalyst bound to diphenylphosphine derivatized polystyrene beads was prepared according to the procedures disclosed in "Supported Transition Metal Complexes as Catalysts", 15 Adv. Organomet. Chem. 189 (1977) (procedure for washing the beads); "[Poly(styryl)bipryidine] palladium(0)-Catalyzed Isomerization of Quadricyclene", 43 J. Org. Chem. 2958 (1978) (procedure for bromination of the beads); "Comparative Study of Some Selected Reactions of Homogeneous and Polymer-Supported Lithium Diorganocuprates", 44 J. Org. Chem. 2705 (1979) (procedure for phosphorylation of the beads); 153 J. Organomet. Chem. 85 (1978), 100 J. Am. Chem. Soc. 7779 (1978) (procedure for loading palladium on the beads). The polystyrene beads, purchased from Polysciences, Inc., Cat. No. 4020 200-400 mesh, were crosslinked with 2 wt% divinylbenzene. The beads were about 37-74 mm in diameter and contained 8.4 wt% phosphine and 4.5 wt% palladium (0.042 mmole). The weight ratio of P to Pd was 6:1. 0.1 g of supported palladium catalyst was swelled in 2.7 ml methylamine in THK (2.8 M) under an argon atmosphere for 1 hour. 0.06 ml (0.75 mmole) of 3,4-epoxy-1-butene was then added. The reaction mixture was stirred at 30° C for 20 hours. The product was analyzed by GC. 2,5- and 2,6-divinyldioxane were not detected in the product. The major products were 4-methylamino-2-buten-1-ol and 2-methylamino-3-butene-1-ol. The regioselectivity of the reaction for the desired 4-methylamino-2-buten-1-ol was 1.8:1. A comparison of this result with Example 8 (unbound catalyst) shows a much higher regioselectivity for the 1,4 adduct when a polymer-bound catalyst is used.

### Example 10 -- Reaction of 3,4-epoxy-1-butene and methylamine catalyzed with polymer bound catalyst

3,4-epoxy-1-butene and methylamine were reacted according to the process described in Example 9, except that the polystyrene beads used as a support were cross-linked with 8 wt% divinylbenzene. The reaction yielded the desired 4-methylamino-2-buten-1-ol and some 2-methylamino-3-butene-1-ol. The regioselectivity of the reaction for the desired 1,4 adduct increased to 2.3:1 as compared to Example 9 where the divinylbenzene concentration was 2 wt% divinylbenzene.

### Examp1e 11 -- Reaction of 3,4-epoxy-1-butene and methylamine catalyzed with polymer bound catalyst

3,4-epoxy-1-butene and methylamine were reacted according to the process described in Example 9, except that the phosphine to palladium ratio on the polystyrene beads was 15. The reaction yielded the desired 4-methylamino-2-buten-1-ol and some 2-methylamino-3-butene-1-ol. The regioselectivity of the reaction for the desired 1,4 adduct was 1.6:1.

### Example 12 -- Reaction of 3,4-epoxy-1-butene and methylamine catalyzed with polymer bound catalyst

3,4-epoxy-1-butene and methylamine were reacted according to the process described in Example 9, except that the polystyrene beads used as a support had a diameter of 15 mm. The reaction yielded the desired 4-methylamino-2-buten-1-ol and some 2-methylamino-3-butene-1-ol. The regioselectivity of the reaction for the desired 1,4 adduct was 1.3:1. This was a decrease compared to Example 9 (1.8:1) where 37-74 mm diameter beads were used.

### Example 13 -- Reaction of 3,4-epoxy-1-butene and methylamine catalyzed with polymer bound catalyst

A palladium catalyst bound to 2,3-O-isopropylidene-2,3-dihydroxy-1,4-bis(diphenylphosphino)butane ("DIOP") derivatized polystyrene beads was prepared according to the procedures described in W. Dumont, "Assymetric Catalytic Reduction with Transition Metal Complexes", 95 J. Am. Chem. Soc. 8295 (1973) (synthesis of DIOP derivatized polystyrene beads); B. M. Trost and E. Keinen, "Steric Steering with Supported Palladium Catalysts", 100 J. Am. Chem. Soc. 7779 (1978) ("Trost") (loading palladium onto beads); and C. U. Pittman and Q. Ng, "Use of Polymer Matrices to Activate Palladium(0) Catalysts and Reduce Catalyst Agglomeration", 153 J. Organomett. Chem. 85 (1978) (loading palladium onto beads). The resulting catalyst contained 3.1 wt% palladium. Methylamine and 3,4-epoxy-1-butene were then reacted in the presence of the synthesized catalyst according to the procedure of Example 9. The regioselectivity of the reaction for the desired 4-methylamino-2-buten-1-ol was 1.5:1.

### Example 14 -- Reaction of 3,4-epoxy-1-butene and methylamine catalyzed with polymer bound catalyst

A palladium catalyst bound to di-para-tolylphosphine derivatized polystyrene beads was prepared. The di-para-tolylphosphine derivatized polystyrene beads were prepared according to the procedure described in Example 9 except that lithium (di-para-tolyl)phosphide was substituted for lithium diphenylphosphide in the procedure for phosphorylation of the polystyrene beads. Palladium was loaded onto the beads by the procedure described in Example 9. The resulting catalyst contained 2.9 wt% palladium. Methylamine and 3,4-epoxy-1-butene were then reacted in the presence of the synthesized catalyst according to the procedure of Example 9. The regioselectivity of the reaction for the desired 4-methylamino-2-buten-1-ol was 1.6:1.

### Example 15 -- Reaction of 3,4-epoxy-1-butene and methylamine catalyzed with polymer bound catalyst

A palladium catalyst bound to tri-2-furylphosphine derivatized chloromethylated polystyrene beads was prepared. Initially, chloromethylated polystyrene beads were crosslinked with 3 wt% divinylbenzene. Next, 3.39 g of tri-2-furylphosphine was dissolved in 80 ml of ether. 5.7 ml of a 2.5 M solution of n-butyl lithium in cyclohexane was added to this solution and the resulting mixture was stirred for two hours. This mixture was transferred to 2 g of chloromethylated polystyrene beads which had been swelled in 20 ml of tetrahydrofuran for one hour. This mixture was stirred for 18 hours, refluxed for one hour, cooled, and quenched with a 3:1 acetone/water mixture. The derivatized chloromethylated polystyrene beads were successively filtered and washed as follows: water (twice), 1:1 acetone/water mixture (once), acetone (twice), dichloromethane (twice), benzene (twice), and methanol (twice). The filtered beads were dried at 50° C and 0.5 mm Hg for 18 hours. Palladium was loaded onto the beads by the procedure described in Example 9. The resulting catalyst contained 3.1 wt% palladium. Methylamine and 3,4-epoxy-1-butene were then reacted in the presence of the synthesized catalyst according to the procedure of Example 9. The regioselectivity of the reaction for the desired 4-methylamino-2-buten-1-ol was 1.4:1.

### Example 16-19 -- Effect of amine concentration on regioselectivity of polymer-bound palladium catalyzed reaction of 3,4-epoxy-1-butene and an amine

3,4-epoxy-1-butene and methylamine were reacted according to the process described in Example 9, except that 0.35 ml (0.43 mmole) of 3,4-epoxy-1-butene was used in each example and the amount of methylamine used was varied to provide the stated methylamine to 3,4-epoxy-1-butene ration. The methylamine was varied by diluting a 6 M methylamine in THF solution by adding additional THF to provide the desired methylamine to 3,4-epoxy-1-butene ration. The experimental details and results are shown in Table I below. "[MeNH₂:EpB]" is the methylamine to 3,4-epoxy-1-butene ratio and "[1,4]:[1,2]" is the regioselectivity of the reaction for the 1,4 adduct.

**TABLE I**

| Example | MeNH₂ (ml) | THF (ml) | [MeNH₂:EpB] | [1,4]:[1,2] |
|---|---|---|---|---|
| 13 | 0.36 | 2.54 | 5:1 | 1.6:1 |
| 14 | 0.73 | 2.18 | 10:1 | 1.8:1 |
| 15 | 1.45 | 1.45 | 20.1 | 1.9:1 |
| 16 | 2.9 | 0 | 40:1 | 2.0:1 |

Table I indicates as the amine concentration increases relative to the epoxide, the regioselectivity of the amine addition for the desired 1,4 adduct increased, as indicated by the ratio of 1,4 to 1,2 adduct formed.

### Example 20 -- Methylamine addition to 3,4-epoxy-1-butene catalyzed by tetrakistriphenylphosphine palladium(0)

A stock solution of methylamine dissolved in tetrahydrofuran ("THF") was prepared by bubbling methylamine into dry, chilled THF with a dry ice-acetone cold trap. The concentration of methylamine in the stock solution was 6M.

A control reaction was performed by dissolving 0.05g of tetrakistriphenylphosphine palladium(0) catalyst in 0.5 ml dry THF under an argon atmosphere. 2 ml of the 6M methylamine-THF stock solution was added to the dissolved catalyst mixture to form a reaction mixture. The air and oxygen were removed by conventional freeze-thaw techniques. 0.035 ml 3,4-epoxy-1-butene was then added to the degassed reaction mixture. The reaction mixture was kept at 30° C for 20 hours. The product of the reaction was analyzed by gas chromatography. The major products of the reaction were a combination of 2,5-divinyl-1,4-dioxane and 2,6-divinyl-1,4-dioxane (60 mole percent). 4-methylamino-2-buten-1-ol (1,4 adduct) and 2-methylamino-3-buten-1-ol (1,2 adduct) were present in a concentration of 23 mole percent of the reaction product. The realtive ratio of 1,4 adduct to 1,2 adduct was 0.4.

### Example 21 -- Methylamine addition to 3,4-epoxy-1-butene catalyzed by [(h3-C3H5)Pd(L)2](BF4) in tetrahydrofuran

Methylamine was reacted with 3,4-epoxy-1-butene in the presence of a catalyst having the general formula [(η³-C₃H₅)Pd(L)₂](BF₄), wherein L is a phosphine ligand. 0.014 g of [(η³-C₃H₅)Pd-(CH₃CN)₂](BF₄) was mixed with a series of phosphine ligands as shown in Table I in 0.2 ml of dry THF under an argon atmosphere to form the catalyst of sample in situ. Each of the ligands shown were purchased from Strem Chemicals, Inc., Newburyport, MA with the exception of P(C₄H₃O)₃ which was prepared according to procedures described in D. Allen et al., "The Chemistry of Heteroarylphosphorus Compounds. Part II. The Importance of Inductive Effects on Pre-equilibria in the Alkaline Hydrolysis of Heteroarylphosphonium Salts; Phosphorus-31 Nuclear Magnetic Resonance Studies," J. Chem. Soc. Perkin II, 63 (1972). 2 ml of a methylamine-THF stock solution prepared according to Example 20 was added to the catalyst mixture to form a reaction mixture. The reaction mixture was degassed by freeze-thaw three times. 0.035 ml 3,4-epoxy-1-butene was then added to the degassed reaction mixture which was kept at 30° C for 20 hours. The reaction product was analyzed by gas chromatography, Table II below illustrates the ratio of the amount of 1,4 adduct formed relative to the amount of the 1,2 adduct formed ("1,4:1,2").

**TABLE II**

| Sample | Ligand | Amount of Ligand(eg.) | 1,4:1,2 |
|---|---|---|---|
| 1 | P(C₆H₅)₃ | 2 | 1.7 |
| 2 | P(C₆H₅)₃ | 4 | 2.4 |
| 3 | P(p-tolyl)₃ | 4 | 2.9 |
| 4 | (C₆H₁₁)(C₆H₅)₂P | 4 | 1.1 |
| 5 | P(C₄H₃₀)₃ | 4 | 1.3 |
| 6 | (C₆H₅)₂P(CH₂)₂P(C₆H₅)₂ | 2 | 2.9 |
| 7 | (C₆H₅)P(CH₂CH₂CN)₂ | 2 | 2.3 |
| 8 | (C₆H₅)₂P(CH₂)₂N(CH₃)₂ | 2 | 1.6 |
| 9 | P(CH₂CH₂PPh₂)₃ | 1 | 2.5 |
| 10 | o-C₆H₄[P(C₆H₅)₂]₂ | 2 | 2.5 |
| 11 | (C₆F₅)₂P(CH₂)₂P(C₆F₅)₂ | 2 | 2.2 |
| 12 | cis-(C₆H₅)₂PCH=CHP(C₆H₅)₂ | 2 | 2.1 |
| 13 | (C₆H₅)₂P(CH₂)₃P(C₆H₅)₂ | 2 | 2.5 |
| 14 | (C₆H₅)₂PCH₂P(C₆H₅)₂ | 2 | 2.2 |

As compared to Example 20 (THF as reaction medium and tetrakistriphenylphosphine palladium(0) as catalyst), the reaction ran without the formation of undesirable products, i.e., no 2,5- or 2,6-divinyl-1,4-dioxane. In addition, the ratio of 1,4-adduct as compared to 1,2-adduct was dramatically increased over the 0.4:1 ratio seen in Example 20.

### Example 22 -- Methylamine addition to 3,4-epoxy-1-butene catalyzed by [(η³-C₃H₅)Pd-((C₆H₅)₂P(m-SO₃C₆H₄Na))₂](BF₄) in liquid methylamine

2 ml of methylamine was condensed into a glass reaction tube equipped with Young's stopcock. The glass tube was capable of holding pressures greater than 2 atmospheres. 0.01 g. of [(η³-C₃H₅)Pd((C₆H₅)₂-P(m-SO₃C₆H₄Na))₂](BF₄) was dissolved in the liquid methylamine. [(C₆H₅)₂P(m-SO₃C₆H₄Na) was synthesized following the procedure by Ahrland et al "The Relative Affinities of Co-ordinating Atoms for Silver Ion. Part II. Nitrogen, Phosphorus, and Arsenic." J. Chem. Soc., 276 (1958)] 0.08 ml 3,4-epoxy-1-butene was added and the reaction solution was stirred at room temperature for four hours and excess methylamine was evaporated. The residue was dissolved in CDCl₃ and nmr analysis was performed. The ratio of 1,4-adduct to 1,2-adduct was 6:1. This ratio was a large increase over the 0.4:1 ratio of 1,4-adduct to 1,2-adduct seen in Example 20.

### Example 23 -- Methylamine addition to 3,4-epoxy-1-butene using water as the reaction medium.

Methylamine was reacted with 3,4-epoxy-1-butene in the presence of various catalysts using water as the reaction medium. Each representative catalyst was dissolved in 0.5 ml water. 1.8 ml of a 40 weight percent solution of methylamine in water was added to the reaction solution, followed by 0.08 ml 3,4-epoxy-1-butene to give an amine to 3,4-epoxy-1-butene molar ratio of 20:1, except in Sample 15, where 2 ml of the methylamine/water solution and 0.35 ml 3,4-epoxy-1-butene were added to increase the ratio to 50:1. In each sample, the molar ratio of catalyst to 3,4-epoxy-1-butene was 0.01:1. The reaction solution was stirred at 30° C for 1.5 hours, concentrated by evaporation, and analyzed by nmr spectrum to determine the 1,4:1,2 ratio. The experimental details and results are shown below in Table III below.

**TABLE III**

| Sample | Catalyst | 1,4:1,2 |
|---|---|---|
| 15 | [(η³-C₃H₅)Pd((C₆H₅)₂P(m-SO₃C₆H₄)Na))₂](BF₄) | 26 |
| 16 | [(η³-C₃H₅)Pd((C₆H₅)₂P(m-SO₃C₆H₄)Na))₂](BF₄) | 15 |
| 17 | [(η³-C₃H₅)PdC1]₂ + 2 eq. (C₆H₅)₂P-(m-SO₃C₆H₄)Na | 15 |
| 18 | Na₂PdC1₄ + 2 eq. (C₆H₅)₂P(m-(SO₃C₆H₄)Na | 14 |
| 19 | [(C₈H₁₂)Pd(CH₃CN)₂](BF₄)** + 2 eq. (C₆H₅)₂P(m-SO₃C₆H₄)Na | 9 |
| 20 | [(η³-C₃H₅)Pd(CH₃CN)₂](BF₄) + 2 eq. P(m-SO₃C₆H₄)₃K₃ | 15 |
| 21 | [(η³-C₃H₅)Pd(CH₃CN)₂](BF₄) + 2 eq. P(C₆H₅)₃* | 7 |
| 22 | [(η³-C₃H₅)Pd(CH₃CN)₂](BF₄) + 2 eq. (C₆H₅)₂P(CH₂)₂P(C₆H₅)₂* | 10 |
| 23 | [(η³-C₃H₅)Pd(CH₃CN)₂](BF₄) + 2 eq. (C₄H₃O)3P* | 9.5 |
| 24 | [(η³-C₃H₅)Pd(CH₃CN)₂](BF₄) + excess P(O-iso-C₃H₇)₃ | 5.3 |
| 25 | Pd(P(C₆H₅)₃)₂C1₂*** | 2.9 |
| 26 | [Pd(CH₃CN)₄](BF₄) + 2 eq. (C₆H₅)₂P(m-SO₃C₆H₄)Na | 2.7 |
| 27 | [Pd(CH₃CN)₄](BF₄) + 4 eq. (C₆H₅)₂P(m-SO₃C₆H₄)Na | 20 |

| | | |
|---|---|---|
| * 0.5ml THF added to dissolve phosphine ligand. | | |
| ** This compound was made by adding [Ag(CH₃CN)₄](BF₄) to (C₈H₁₂)PDC1₂ (in 2 to 1 ratio) in dichloromethane. The AgC1 precipitate was filtered and the filtrate was concentrated to dryness. C₈H₁₂ is 1,5-cyclooctadiene. | | |
| *** Not soluble in water. | | |

This series of samples shows increased 1,4:1,2 ratios as compared to the preceding examples. This series of samples indicates that high 1,4:1,2 ratios can be obtained by using a highly polar reaction medium (water) and an appropriate catalyst.
Furthermore, as shown by Samples 21, 22, and 23, a mixture of water and a less polar liquid such as THF can also obtain high 1,4:1,2 ratios. A comparison of Samples 2 and 5 (THF alone as reaction medium with Samples 21 and 23 (mixture of water and THF), respectively, illustrate that a reaction medium comprising a mixture of water and THF produces higher 1,4:1,2 ratios than THF used alone.

### Example 24 -- Methylamine addition to 3,4-epoxy-1-butene using methanol as the reaction medium

Methylamine was reacted with 3,4-epoxy-1-butene in methanol as a reaction medium. The catalyst was [(h³-C₃H₅)Pd(Ph₂P(m-SO₃C₆H₄Na))₂] (BF₄). 0.012 g of catalyst was added to 2 ml of a methylamine in methanol stock solution prepared according to Example 20 (11.5 M). 0.08 ml 3,4-epoxy-1-butene was then added to the solution and the reaction mixture as heated at 30° C for 1.5 hours. The reaction product was analyzed by NMR. The selectivity (1,4:1,2) was determined to be 5:1.

### Example 25 -- Methylamine addition to 3,4-epoxy-1-butene using methylamine in acetonitrile as the reaction medium

Methylamine was reacted with 3,4-epoxy-1-butene according to the procedure of Example 24 except that methylamine in acetonitrile (11.4 M) was used as the reaction medium. The selectivity (1,4:1,2) was determined to be 4:1.

### Example 26 -- Methylamine addition to 3,4-epoxy-1-butene using toluene as the reaction medium

Methylamine was reacted with 3,4-epoxy-1-butene in toluene as a reaction medium. The catalyst included 0.01 g of [(η³-C₃H₅)Pd(CH₃CN)₂](BF₄) and 0.0166 g PPh₃ were mixed in 0.2 mL CH₂Cl₂. Methylamine in toluene (2.5 ml, 4.8 M) was added. The mixture was frozen and 0.04 ml 3,4-epoxy-1-butene was added. The reaction mixture was kept at 30° C for 16 hours. The reaction product was analyzed by gas chromatography. The 1,4 to 1,2 ratio was 3.

### Example 27 -- Primary amine addition to 3,4-epoxy-1-butene using water as the reaction medium

Various primary amines were reacted with 3,4-epoxy-1-butene according to the procedure described in Example 3, substituting ethylamine (EtNH₂), cyclohexylamine (C₆H₁₁NH₂), ammonia (NH₃), and ethanolamine (H₂NC₂H₅OH) for methylamine. The ratio of primary amine to 3,4-epoxy-1butene was 20:1, except in the case of C₆H₁₁NH₂, in which the ratio of primary amine to 3,4-epoxy-1-butene was 14:1. The results are shown in Table IV below.

**TABLE IV**

| AMINE | [1,4:1,2] |
|---|---|
| CH₃CH₂NH₂ | 25 |
| C₆H₁₁NH₂ | 14 |
| NH₃ | 6 |
| H₂NC₂H₅OH | 5 |

Table IV indicates that the use of water as the reaction medium results in high yields of 1,4-adduct with a variety of primary amines.

## Claims

1. A process of forming 4-amino-substituted-2-buten-1-ol comprising the steps of:
providing a quantity of 3,4-epoxy-1-butene;
providing a quantity of an amine; and
reacting said quantity of 3,4-epoxy-1-butene with said amine in a reaction medium and in the presence of a catalyst consisting essentially of a complex of palladium with up to four phosphine ligands under conditions effective to form 4-amino-substituted-2-buten-1-ol.

2. A process according to claim 1, wherein said amine is selected from the group consisting of primary amines, secondary amines, cyclin amines, and heterocyclic amines.

3. A process according to Claim 1, wherein said complex of palladium is bound to a polymer selected from the group consisting of polystyrene, polystyrene beads crosslinked with divinylbenzene, polyvinyl chloride, polyvinyl alcohol, polybutadiene, alumina, cellulose, silica gel, and a copolymer of styrene, methacrylate, and ethylene 1,2-dimethacrylate.

4. A process according to Claim 1, wherein said reaction medium is a solvent selected from the group consisting of tetrahydrofuran, diethyl ether, toluene, benzene, N,N-dimethylformamide, dimethyl sulfoxide, dioxane, ethyl alcohol, dichloromethane, and 1,2-dimethoxyethane.

5. A process according to Claim 1, wherein said amine is present in said solvent in a range from about 0.1 to 10.0 M and in a molar ratio relative to said 3,4-epoxy-1-butene in the range from about 3:1 to 40:1, and the quantity of said catalyst is from about 0.1 to about 10 mole percent relative to the quantity of said 3,4-epoxy-1-butene.

6. A process for forming 4-amino-2-buten-1-ol, comprising the steps of:
providing a quantity of 3,4-epoxy-1-butene;
providing a quantity of primary amine; and
reacting said quantity of 3,4-epoxy-1-butene with said quantity of primary amine in a reaction medium and in the presence of a catalyst, wherein said catalyst is a complex of palladium and phosphine ligands and said reaction medium is a liquid having an E_{T}(30) no less than about 32.

7. A process according to claim 6, wherein said liquid is selected from the group consisting of toluene, benzene, diethylether, 1,4-dioxane, tetrahydrofuran, and chloroform.

8. A process according to claim 6, wherein said primary amine is selected from the group consisting of ammonia, methylamine, ethylamine, propylamine, butylamine, hexylamine, octylamine, decylamine, allylamine, 2-hexenylamine, 4-decenylamine, ethanolamine, octanolamine, aniline, and cyclohexylamine.

9. A process according to claim 6, wherein said liquid is selected from the group consisting of acetone, dimethylsulfoxide, acetonitrile, nitromethane, 2-propanol, 2-methoxyethanol, N-methylformamide, methanol, 1,2-ethanediol, glycerol, ethanol, 2,2,2-trifluoroethanol, water, and an 80:20 solution of ethanol and water.

10. A method according to claim 6, wherein said catalyst is selected from the group consisting of:
Pd(PR₁R₂R₃)₂X₂;
Pd(R₄R₅P(V)_{b}ZR₆R₇)X₂;
[(η³-CH(R₈)C(R₉)CH(R₁₀))Pd(PR₁R₂R₃)₂]X;
[(η³-CH(R₈)C(R₉)CH(R₁₀))Pd(R₄R₅P(V)_{b}ZR₆R₇)]X;
Pd(R₄R₅P(V)_{b}ZR₆R₇)₂;
Pd(PR₁R₂R₃)₄;
[Pd(D)(PR₁R₂R₃)₂]X₂; and
[Pd(D)R₄R₅(V)_{b}ZR₆R₇)]X₂;
wherein
R₁, R₂, R₃, R₄, R₅, R₆, and R₇ can be the same or different and each are an alkyl, cycloalkyl, alkoxide, aryl, heterocyclic, functionalized alkyl, functionalized aryl, or ether group;
R₈, R₉, and R₁₀ are the same or different and each are hydrogen, alkyl, cycloalkyl, aryl, heterocyclic, halogen, ether, sulfide, carbonyl or are linked together to form a ring;
V is CH₂ or CH as part of unsaturated component;
Z is phosphorus or nitrogen;
D is a cyclic diene;
X is an anion;
Ph is a phenyl group;
P is phosphorus;
Pd is palladium; and
b is a number in the range from 1 to 4.

11. A method according to claim 6, wherein said liquid is water or a mixture of liquids including water and said catalyst is selected from the group consisting of:
Pd(Ph₂P(m-C₆H₄SO₃Q))₂X₂
Pd(P(m-C₆H₄SO₃Q)₃)₂X₂
Pd(Ph₂P(o-CO₂C₆H₄Q))₂X₂
Pd(P(CH₂CH₂(CH₂CH₂O)ₐCH₃)₃)₂X₂
Pd(PhP(CH₂CH₂CH₂OCH₃)₂)₂X₂
Pd(Ph₂PCH₂CH₂N(CH₃)₃X)₂X₂
Pd(Ph₂PCH₂CH₂CO₂Q)X₂
Pd[(C₆H₅)₂P(CH₂)₂N(CH₃)₂]X₂
Pd[(m-SO₃C₆H₄Q)c(C₆H₅)_{2-c}P(CH₂)_{d}P(C₆H₅)_{f}(m-SO₃C₆H₄Q)-_{2-f}]X₂
Pd[(m-SO₃C₆H₄Q)_{c}(C₆H₅)_{2-c}PCH(CH₃)CH(CH₃)P(C₆H₅) _{f}(m-SO₃C₆H₄Q)_{2-f}]X₂
Pd[(p-(CH₃)₃NC₆H₄X)₂PC₂H₄P(p-(CH₃)₃NC₆H₄X)₂]X₂
Pd[(p-(CH₃)₃NC₆H₄X)₂PCH(CH₃)CH(CH₃)P-(p-(CH₃)₃NC₆H₄X)₂]X₂
and 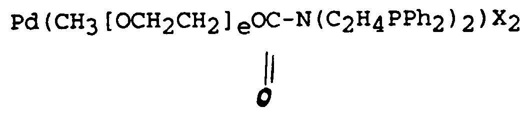
[(η³-C₃H₅)Pd(P(m-C₆H₄SO₃Q)₃)₂]X
[(η3-C₃H₅)Pd(Ph₂P(m-C₆H₄SO₃Q))₂]X
[(η³-C₃H₅)Pd(Ph₂P(m-C₆H₄CO₂Q))₂]X
[(η³-C₃H₅)Pd(P(CH₂(CH₂CH₂O)ₐCH₃)₃)₂]X
[(η³-C₃H₅)Pd(PhP(CH₂CH₂CH₂OCH₃)₂)₂]X
[(η³-C₃H₅)Pd(Ph₂PCH₂CH₂N(CH₃)₃X)₂]X
[(η³-C₃H₅)Pd(P(O-iso-C₃H₇)₃)₂]X
[(η³-C₃H₅)Pd[(m-SO₃C₆H₄Q)_{c}(C₆H₅)_{2-c}P(CH₂)_{d}P(C₆H₅) _{f}(m-SO₃C₆H₄Q))_{2-f}]X
[(η³-C₃H₅)Pd(p-(CH₃)₃NC₆H₄X)₂PC₂H₄P(p-(CH₃)₃NC₆H₄X)₂]X
[(η³-C₃H₅)Pd(p-(CH₃)₃NC₆H₄X)₂PCH(CH₃)CH(CH₃)P-(p-(CH₃)₃NC₆H₄X)₂]X
Pd[(C₆H₅)₂P(CH₂)₂N(CH₃)₂]₂
Pd(P(m-C₆H₅SO₃Q)₃)₄
Pd[Ph₂P(m-SO₃C₆H₄Q)]₃
Pd[Ph₂P(m-SO₃C₆H₄Q)]₄
Pd[P(CH₂(CH₂CH₂O)ₐCH₃)₃]₄
Pd[P(CH(CH₃)(CH₂CH₂O)₃CH₃)₃]₄
Pd[PhP(CH₂CH₂CH₂OCH₃)₂]₄
Pd[Ph₂PCH₂CH₂N(CH₃)₃X]₄
Pd[p-(CH₃)₃NC₆H₄X)₂PCH(CH₃)CH(CH₃)P(p-(CH₃)₃NC₆H₄X)₂]₂
Pd[(p-(CH₃)₃NC₆H₄X)₂PC₂H₄P(p-(CH₃)₃NC₆H₄X)₂]₂
and
Pd[(m-SO₃C₆H₄Q)_{c}(C₆H₅)_{2-c}P(CH₂)_{d}P(C₆H₅)-_{f}(m-SO₃C₆H₄Q)_{2-f}]₂
[(C₈H₁₂)Pd(P(m-C₆H₄SO₃Q)₃)₂]X₂
[(C₇H₈)Pd(P(CH₂(CH₂CH₂O)ₐCH₃)₃)₂]X₂
[(C₇H₈)Pd(PhP(CH₂CH₂CH₂OCH₃)₂)₂]X₂
[(C₇H₈)Pd(Ph₂PCH₂CH₂N(CH₃)₃X)₂]X₂
[(C₇H₈)Pd((C₆H₅)₂P(m-SO₃C₆H₄Q))₂]X₂
[(C₇H₈)Pd(P(m-SO₃C₆H₄Q)₃)₂]X₂
[(C₈H₁₂)Pd(P(CH₂(CH₂(CH₂CH₂O)ₐCH₃)₃)₂]X₂
[(C₈H₁₂)Pd(PhP(CH₂CH₂CH₂OCH₃)₂)₂]X₂
[(C₇H₈)Pd((p-(CH₃)₃NC₆H₄X)₂PCH(CH₃)CH(CH₃)P-(p-(CH₃)₃NC₆H₄X)₂]X₂
[(C₇H₈)Pd(m-SO₃C₆H₄Q)_{c}(C₆H₅)_{2-c}P(CH₂)_{d}P(C₆H₅) f(m-SO₃C₆H₄Q)_{2-f}]X₂
[(C₇H₈)Pd(p-(CH₃)₃NC₆H₄X)₂PC₂H₄P(p-(CH₃)₃NC₆H₄X)₂]X₂ 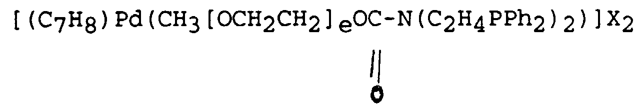
[(C₇H₈)Pd(m-SO₃C₆H₄Q)c(C₆H₅)_{2-c}PCH(CH₃)CH(CH₃)P(C₆H₅) _{f}(m-SO₃C₆H₄Q)_{2-f}]X₂[(C₈H₁₂)Pd((p-
(CH₃)₃NC₆H₄X)₂PCH(CH₃)CH(CH₃)P (p-(CH₃)₃NC₆H₄X)₂]X₂
[(C₈H₁₂)Pd(p-(CH₃)₃NC₆H₄X)₂PC₂H₄P(p-(CH₃)₃NC₆H₄X)₂]X₂ 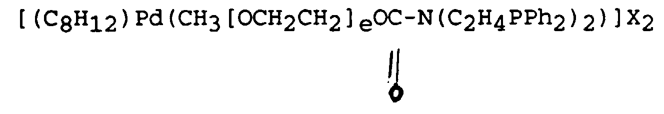
[(C₈H₁₂)Pd(m-SO₃C₆H₄Q)_{c}(C₆H₅)_{2-c}P(CH₂)_{d}P(C₆H₅)-_{c}(m-SO₃C₆H₄Q)_{2-c}]X₂
[(C₈H₁₂)Pd(p-(CH₃)₃NC₆H₄X)₂PC₂H₄P(p-(CH₃)₃NC₆H₄X)₂]X₂
[(C₈H₁₂)Pd(m-SO₃C₆H₄Q)_{c}(C₆H₅)_{2-c}PCH(CH₃)CH(CH₃)P(C₆H₅)-_{f}(m-SO₃C₆H₄Q)_{2-f}]X₂
wherein Ph is a phenyl group P is phosphorus, Pd is palladium, a is a number in the range from 1 to 3, b is a number in the range from 1 to 4, c and f are 1 or 2, d is a number in the range from 2 to 4, e is 12 or 16, Q is a cation, and X is an anion.

## Patentansprüche

1. Verfahren zur Bildung von 4-Amino-substituiertem 2-Buten-1-ol, umfassend die Schritte:
Vorlegen einer Quantität an 3,4-Epoxy-1-buten,
Vorlegen einer Quantität eines Amins und
Umsetzen der Quantität an 3,4-Epoxy-1-buten mit dem Amin in einem Reaktionsmedium und in Gegenwart eines Katalysatoren, der im wesentlichen einen Palladiumkomplex mit bis zu vier Phosphinliganden umfaßt, unter zur Bildung von 4-Amino-substituiertem 2-Buten-1-ol wirksamen Bedingungen.

2. Verfahren nach Anspruch 1, worin das Amin ausgewählt ist aus der Gruppe bestehend aus primären Aminen, sekundären Aminen, cyclischen Aminen und heterocyclischen Aminen.

3. Verfahren nach Anspruch 1, worin der Palladiumkomplex an ein Polymer gebunden ist, ausgewählt aus der Gruppe bestehend aus Polystyren, Polystyrenperlen quervernetzt mit Divinylbenzen, Polyvinylchlorid, Polyvinylalkohol, Polybutadien, Alumina, Cellulose, Silikagel und einem Copolymer aus Styren, Methacrylat und Ethylen-1,2-Dimethacrylat.

4. Verfahren nach Anspruch 1, worin das Reaktionsmedium ein Lösemittel ist, ausgewählt aus der Gruppe bestehend aus Tetrahydrofuran, Diethyläther, Toluen, Benzen, N-N-Dimethylformamid, Dimethylsulfoxid, Dioxan, Ethylalkohol, Dichlormethan und 1,2-Dimethoxyethan.

5. Verfahren nach Anspruch 1, worin das Amin in dem Lösemittel in einem Bereich von etwa 0,1 bis 10,0 M und in einem relativen Molverhältnis zu 3,4-Epoxy-1-buten im Bereich von etwa 3:1 bis 40:1 vorhanden ist, und die Katalysatormenge zwischen etwa 0,1 bis etwa 10 Mol-%, bezogen auf die Quantität an 3,4-Epoxy-1-buten, liegt.

6. Verfahren zur Bildung von 4-Amino-2-buten-1-ol, umfassend die Schritte:
Vorlegen einer Quantität an 3,4-Epoxy-1-buten,
Vorlegen einer Quantität an primärem Amin und
Umsetzen der Quantität an 3,4-Epoxy-1-buten
mit der Quantität an primärem Amin in einem Reaktionsmedium und in Gegenwart eines Katalysatoren, wobei der Katalysator ein Komplex aus Palladium und Phosphinliganden ist und das Reaktionsmedium eine Flüssigkeit ist, die einen E_{T}(30)-Wert von weniger als etwa 32 hat.

7. Verfahren nach Anspruch 6, worin die Flüssigkeit ausgewählt ist aus der Gruppe bestehend aus Toluen, Benzen, Diethylether, 1,4-Dioxan, Tetrahydrofuran und Chloroform.

8. Verfahren nach Anspruch 6, worin das primäre Amin ausgewählt ist aus der Gruppe bestehend aus Ammoniak, Methylamin, Ethylamin, Propylamin, Butylamin, Hexylamin, Octylamin, Decylamin, Allylamin, 2-Hexenylamin, 4-Decenylamin, Ethanolamin, Octanolamin, Anilin und Cyclohexylamin.

9. Verfahren nach Anspruch 6, worin die Flüssigkeit ausgewählt ist aus der Gruppe bestehend aus Aceton, Dimethylsulfoxid, Acetonnitril, Nitromethan, 2-Propanol, 2-Methoxyethanol, N-Methylformamid, Methanol, 1,2-Ethandiol, Glycerol, Ethanol, 2,2,2-Trifluorethanol, Wasser und eine 80:20-Lösung aus Ethanol und Wasser.

10. Verfahren nach Anspruch 6, worin der Katalysator aus gewählt ist aus der Gruppe bestehend aus:
Pd(PR₁R₂R₃)₂X₂;
Pd(R₄R₅P(V)_{b}ZR₆R₇)X₂;
(η³-CH(R₈)C(R₉)CH(R₁₀))Pd(PR₁R₂R₃)₂]X;
[(η³-CH(R₈)C(R₉)CH(R₁₀))Pd(R₄R₅P(V)_{b}ZR₆R₇)]X;
Pd(R₄R₅P(V)_{b}ZR₆R₇)₂;
Pd(PR₁R₂R₃)₄;
[Pd(D)(PR₁R₂R₃)₂]X₂; and
[Pd(D)R₄R₅(V)_{b}ZR₆R₇)]X₂;
worin R₁, R₂, R₃, R₄, R₅, R₆ und R₇ gleich oder verschieden sein können und jeweils eine Alkyl-, Cycloalkyl-, Alkoxid-, Aryl-, Heterocyclo-, funktionalisierte Alkyl-, funktionalisierte Aryl- oder Ethergruppe;
R₈, R₉ und R₁₀ sind gleich oder verschieden und jeweils Wasserstoff, Alkyl, Cycloalkyl, Aryl, heterocyclisch, Halogen, Ether, Sulfid, Carbonyl oder unter Bildung eines Rings miteinander verknüpft;
V ist CH₂ oder CH als Teil eines ungesättigten Bestandteils,
Z ist Phosphor oder Stickstoff,
D ist ein cyclisches Dien,
X ist ein Anion, Ph ist eine Phenylgruppe,
P ist Phosphor,
Pd ist Palladium und
b ist eine Zahl im Bereich von 1 bis 4.

11. Verfahren nach Anspruch 6, worin die Flüssigkeit Wasser oder eine Mischung aus Flüssigkeiten einschließlich Wasser ist und der Katalysator ausgewählt ist aus der Gruppe bestehend aus:
Pd(Ph₂P(m-C₆H₄SO₃Q))₂X₂
Pd(P(m-C₆H₄SO₃Q)₃)₂X₂
Pd(Ph₂P(o-CO₂C₆H₄Q))₂X₂
Pd(P(CH₂CH₂(CH₂CH₂O)ₐCH₃)₃)₂X₂
Pd(PhP(CH₂CH₂CH₂OCH₃)₂)₂X₂
Pd(Ph₂PCH₂CH₂N(CH₃)₃X)₂X₂
Pd(Ph₂PCH₂CH₂CO₂Q)X₂
Pd[(C₆H₅)₂P(CH₂)₂N(CH₃)₂]X₂
Pd[(m-SO₃C₆H₄Q)_{c}(C₆H₅)_{2-c}P(CH₂)_{d}P(C₆H₅)_{f}(m-SO₃C₆H₄Q)-_{2-f}]X₂
Pd[(m-SO₃C₆H₄Q)_{c}(C₆H₅)_{2-c}PCH(CH₃)CH(CH₃)P(C₆H₅) _{f}(m-SO₃C₆H₄Q)_{2-f}]X₂
Pd[(p-(CH₃)₃NC₆H₄X)₂PC₂H₄P(p-(CH₃)₃NC₆H₄X)₂]X₂
Pd[(p-(CH₃)₃NC₆H₄X)₂PCH(CH₃)CH(CH₃)P-(p-(CH₃)₃NC₆H₄X)₂]X₂
und 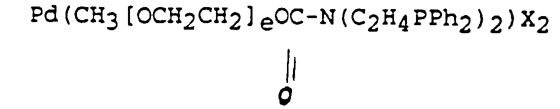
[(η³-C₃H₅)Pd(P(m-C₆H₄SO₃Q)₃)₂]X
[(η³-C₃H₅)Pd(Ph₂P(m-C₆H₄SO₃Q))₂]X
[(η³-C₃H₅)Pd(Ph₂P(m-C₆H₄CO₂Q))₂]x
[(η³-C₃H₅)Pd(P(CH₂(CH₂CH₂O)ₐCH₃)₃)₂]X
[(η³-C₃H₅)Pd(PhP(CH₂CH₂CH₂OCH₃)₂)₂]X
[(η³-C₃H₅)Pd(Ph₂PCH₂CH₂N(CH₃)₃X)₂]X
[(η³-C₃H₅)Pd(P(O-iso-C₃H₇)₃)₂]X
[(η³-C₃H₅)Pd[(m-SO₃C₆H₄Q)_{c}(C₆H₅)_{2-c}P(CH₂)_{d}P(C₆H₅) _{f}(m-SO₃C₆H₄Q))_{2-f}]X
[(η³-C₃H₅)Pd(p-(CH₃)₃NC₆H₄X)₂PC₂H₄P(p-(CH₃)₃NC₆H₄X)₂]X
[(η³-C₃H₅)Pd(p-(CH₃)₃NC₆H₄X)₂PCH(CH₃)CH(CH₃)P-(p-(CH₃)₃NC₆H₄X)₂]X
Pd[(C₆H₅)₂P(CH₂)₂N(CH₃)₂]₂
Pd(P(m-C₆H₄SO₃Q)₃)₄
Pd[Ph₂P(m-SO₃C₆H₄Q)]₃
Pd[Ph₂P(m-SO₃C₆H₄Q)]₄
Pd[P(CH₂(CH₂CH₂O)ₐCH₃)₃]₄
Pd[P(CH(CH₃)(CH₂CH₂O)₃CH₃)₃]₄
Pd[PhP(CH₂CH₂CH₂OCH₃)₂]₄
Pd[Ph₂PCH₂CH₂N(CH₃)₃X]₄
Pd[p-(CH₃)₃NC₆H₄X)₂PCH(CH₃)CH(CH₃)P(p-(CH₃)₃NC₆H₄X)₂]₂
Pd[(p-(CH₃)₃NC₆H₄X)₂PC₂H₄P(p-(CH₃)₃NC₆H₄X)₂]₂
und
Pd[(m-SO₃C₆H₄Q)_{c}(C₆H₅)_{2-c}P(CH₂)_{d}P(C₆H₅)-_{f}(m-SO₃C₆H₄Q)_{2-f}]₂
[(C₈H₁₂)Pd(P(m-C₆H₄SO₃Q)₃)₂]X₂
[(C₇H₈)Pd(P(CH₂(CH₂CH₂O)ₐCH₃)₃)₂]X₂
[(C₇H₈)Pd(PhP(CH₂CH₂CH₂OCH₃)₂)₂]X₂
[(C₇H₈)Pd(Ph₂PCH₂CH₂N(CH₃)₃X)₂]X₂
[(C₇H₈)Pd((C₆H₅)₂P(m-SO₃C₆H₄Q))₂]X₂
[(C₇H₈)Pd(P(m-SO₃C₆H₄Q)₃)₂]X₂
[(C₈H₁₂)Pd(P(CH₂(CH₂(CH₂CH₂O)ₐCH₃)₃)₂]X₂
[(C₈H₁₂)Pd(PhP(CH₂CH₂CH₂OCH₃)₂)₂]X₂
[(C₇H₈)Pd((p-(CH₃)₃NC₆H₄X)₂PCH(CH₃)CH(CH₃)P-(p-(CH₃)₃NC₆H₄X)₂]X₂
[(C₇H₈)Pd(m-SO₃C₆H₄Q)_{c}(C₆H₅)_{2-c}P(CH₂)_{d}P(C₆H₅) f(m-SO₃C₆H₄Q)_{2-f}]X₂
[(C₇H₈)Pd(p-(CH₃)₃NC₆H₄X)₂PC₂H₄P(p-(CH₃)₃NC₆H₄X)₂]X₂ 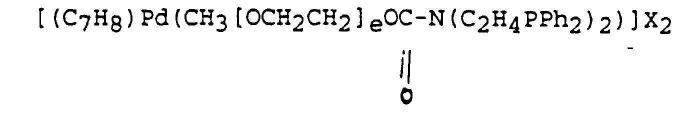
[(C₇H₈)Pd(m-SO₃C₆H₄Q)_{c}(C₆H₅)_{2-c}PCH(CH₃)CH(CH₃)P(C₆H₅) _{f}(m-SO₃C₆H₄Q)_{2-f}]X₂[(C₈H₁₂)Pd((p-
(CH₃)₃NC₆H₄X)₂PCH(CH₃)CH(CH₃)P (p-(CH₃)₃NC₆H₄X)₂]X₂
[(C₈H₁₂)Pd(p-(CH₃)₃NC₆H₄X)₂PC₂H₄P(p-(CH₃)₃NC₆H₄X)₂]X₂ 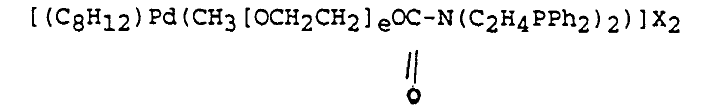
[(C₈H₁₂)Pd(m-SO₃C₆H₄Q)_{c}(C₆H₅)_{2-c}P(CH₂)_{d}P(C₆H₅)-_{c}(m-SO₃C₆H₄Q)_{2-c}]X₂
[(C₈H₁₂)Pd(p-(CH₃)₃NC₆H₄X)₂PC₂H₄P(p-(CH₃)₃NC₆H₄X)₂]X₂
[(C₈H₁₂)Pd(m-SO₃C₆H₄Q)_{c}(C₆H₅)_{2-c}PCH(CH₃)CH(CH₃)P(C₆H₅)-_{f}(m-SO₃C₆H₄Q)_{2-f}]X₂
worin Ph eine Phenylgruppe ist, P ist Phosphor, Pd ist Palladium, a ist eine Zahl im Bereich von 1 bis 8, b ist eine Zahl im Bereich von 1 bis 4, c und f sind 1 oder 2, d ist eine Zahl im Bereich von 2 bis 4, e ist 12 oder 16, Q ist ein Kation und X ist ein Anion.

## Revendications

1. Procédé pour former le 2-butène-1-ol 4-amine-substitué comprenant les étapes de :
fournir une quantité de 3,4-époxy-1-butène ;
fournir une quantité d'une amine ; et
réagir ladite quantité de 3,4-époxy-1-butène avec ladite amine dans un milieu réactionnel et en présence d'un catalyseur consistant essentiellement en un complexe de palladium avec jusqu'à quatre ligands de phosphine sous des conditions efficaces pour la formation de 2-butène-1-ol 4-amine-substitué.

2. Procédé selon la revendication 1, dans lequel ladite amine est choisie parmi le groupe consistant en les suivantes : une amine primaire, une amine secondaire, une amine cyclique, et une amine hétérocyclique.

3. Procédé selon la revendication 1, dans lequel ledit complexe de palladium est lié à un polymère choisi parmi le groupe consistant en les suivants : polystyrène, les grains de polystyrène réticulés avec le divinylbenzène, chlorure de polyvinyle, alcool de polyvinyle, polybutadiène, alumine, cellulose, gel de silice, et un copolymère de styrène, de métacrylate, et 1,2-diméthacrylate d'éthylène.

4. Procédé selon la revendication 1, dans lequel ledit milieu réactionnel est un solvant choisi parmi le groupe consistant en les suivants : tétrahydrofurane, diéthyléther, toluène, benzène, N,N'-diméthylformamide, diméthylsulfoxide, dioxane, alcool éthylique, dichlorométhane, et 1,2-diméthoxyéthane.

5. Procédé selon la revendication 1 dans lequel ladite amine est présente dans ledit solvant d'environ 0,1 à 10,0 M et d'un rapport molaire relatif audit 3,4-époxy-1-butène d'environ 3:1 à 40:1, et la quantité dudit catalyseur est d'environ 0,1 à environ 10 mol pour-cent relative à la quantité dudit 3,4-époxy-1-butène.

6. Procédé pour former le 4-amino-2-butène-1-ol, comprenant les étapes de :
fournir une quantité de 3,4-époxy-1-butène ;
fournir une quantité d'une amine primaire ; et
réagir ladite quantité de 3,4-époxy-1-butène avec ladite quantité de l'amine primaire dans un milieu réactionnel et en présence d'un catalyseur, dans lequel ledit catalyseur est un complexe de palladium et de ligands de phosphine et ledit milieu réactionnel est un liquide ayant une valeur de E_{T}(30) de pas moins d'environ 32.

7. Procédé selon la revendication 6, dans lequel ledit liquide est choisi parmi le groupe consistant en les suivants : toluène, benzène, diéthyléther, 1,4-dioxane, tétrahydrofurane, et chloroforme.

8. Procédé selon la revendication 6, dans lequel ladite amine primaire est choisie parmi le groupe consistant en les suivantes : ammoniac, méthylamine, éthylamine, propylamine, butylamine, hexylamine, octylamine, décylamine, allylamine, 2-hexanylamine, 4-décénylamine, éthanolamine, octanolamine, aniline, et cyclohexylamine.

9. Procédé selon la revendication 6, dans lequel ledit liquide est choisi parmi le groupe consistant en les suivants : acétone, diméthylsulfoxide, acétonitrile, nitrométhane, 2-propanol, 2-méthoxyéthanol, N-méthylformamide, méthanol, 1,2-éthanediol, glycérol, éthanol, 2,2,2-trifluoroéthanol, eau, et une solution 80 : 20 d'éthanol et eau.

10. Méthode selon la revendication 6, dans laquelle le dit catalyseur est choisi parmi le groupe consistant en :
Pd(PR₁R₂R₃)₂X₂ ;
Pd(R₄R₅P(V)_{b}ZR₆R₇)X₂ ;
[(η³-CH(R₈)C(R₉)CH(R₁₀))Pd(PR₁R₂R₃)₂]X ;
[(η³-CH(R₈)C(R₉)CH(R₁₀))Pd(R₄R₅P(V)_{b}ZR₆R₇)]X ;
Pd(R₄R₅P(V)_{b}ZR₆R₇)₂ ;
Pd(PR₁R₂R₃)₄ ;
[Pd(D)(PR₁R₂R₃)₂]X₂ ; et
[Pd(D)R₄R₅(V)_{b}ZR₆R₇)]X₂ ;
dans lequel
R₁, R₂, R₃, R₄, R₅, R₆, et R₇ peuvent être identiques ou différents et chacun sont un groupe alkyle, cycloalkyle, alkoxyde, aryle, hétérocyclique, alkyle fonctionnalisé, aryle fonctionnalisé, ou un groupe éther ;
R₈, R₉, et R₁₀ sont identiques ou différents et chacun est hydrogène, alkyle, cycloalkyle, aryle, hétérocyclique, halogène, éther, sulfure, carbonyle ou sont attachés ensemble pour former un cycle ;
V est CH₂ ou CH faisant partie d'un composant insaturé ;
Z est un atome de phosphore ou d'azote ;
D est diène cyclique ;
X est un anion ;
Ph est un groupe phényle ;
P est atome de phosphore;
Pd est un atome de palladium ; et
b est un numéro de 1 à 4.

11. Méthode selon la revendication 6, dans laquelle ledit liquide est de l'eau ou un mélange de liquides comprenant de l'eau et ledit catalyseur est choisi parmi le groupe consistant en les suivants :
Pd(Ph₂P(m-C₆H₄SO₃Q))₂X₂
Pd(P(m-C₆H₄SO₃Q)₃)₂X₂
Pd(Ph₂P(o-CO₂C₆H₄Q))₂X₂
Pd(P(CH₂CH₂(CH₂CH₂O)ₐCH₃)₃)₂X₂
Pd(PhP(CH₂CH₂CH₂OCH₃)₂)₂X₂
Pd(Ph₂PCH₂CH₂N(CH₃)₃X)₂X₂
Pd(Ph₂PCH₂CH₂CO₂Q)X₂
Pd[(C₆H₅)₂P(CH₂)₂N(CH₃)₂]X₂,
Pd[(m-SO₃C₆H₄Q)_{c}(C₆H₅)₂P(CH₂)_{d}P(C₆H₅)_{f}(m-SO₃C₆H₄Q)_{2-f}]X₂
Pd[(m-SO₃C₆H₄Q)_{c}(C₆H₅)_{2-c}PCH(CH₃)CH(CH₃)P(C₆H₅)_{f}(m-SO₃C₆H₄Q)_{2-f}]X₂
Pd[p-(CH₃)₃NC₆H₄X)₂PC₂H₄P(p-(CH₃)₃NC₆H₄X)₂]X₂
Pd[(p-(CH₃)₃NC₆H₄X)₂PCH(CH₃)CH(CH₃)P(p-(CH₃)₃-NC₆H₄X)₂]X₂
et 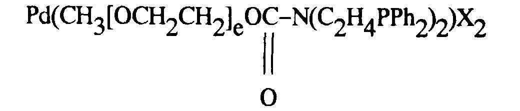
[(η³-C₃H₅)Pd(P(m-C₆H₄SO₃Q)₃)₂]X
[(η³-C₃H₅)Pd(Ph₂P(m-C₆H₄SO₃Q))₂]X
[(η³-C₃H₅)Pd(Ph₂P(m-C₆H₄CO₂Q))₂]X
[(η³-C₃H₅)Pd(P(CH₂CH₂O)ₐCH₃)₃)₂]X
[(η³-C₃H₅)Pd(PhP(CH₂CH₂CH₂OCH₃)₂)₂]X
[(η³-C₃H₅)Pd(Ph₂PCH₂CH₂N(CH₃)₃X)₂]X
[(η³-C₃H₅)Pd(P(O-iso-C₃H₇)₃)₂]X
[(η³-C₃H₅)Pd(m-SO₃C₆H₄Q)_{c}(C₆H₅)₂P(CH₂)_{d}P(C₆H₅)_{f}(m-SO₃C₆H₄Q))_{2-f}]X
[(η³-C₃H₅)Pd(p-(CH₃)₃NC₆H₄X)₂PC₂H₄P(p-(CH₃)₃NC₆H₄X)₂]X
[(η³-C₃H₅)Pd(p-(CH₃)₃NC₆H₄X)₂PCH(CH₃)CH(CH₃)P(p-(CH₃)₃NC₆H₄X)₂]X
Pd[(C₆H₅)₂P(CH₂)₂N(CH₃)₂]₂
Pd(P(m-C₆H₄SO₃Q)₃)₄
Pd[Ph₂P(m-SO₃C₆H₄Q)]₃
Pd[Ph₂P(m-SO₃C₆H₄Q)]₄
Pd[P(CH₂(CH₂CH₂O)ₐCH₃)₃]₄
Pd[P(CH(CH₃)(CH₂CH₂O)₃CH₃)₃]₄
Pd[PhP(CH₂CH₂CH₂OCH₃)₂]₄
Pd[Ph₂PCH₂CH₂N(CH₃)₃X]₄
Pd[p-(CH₃)₃NC₆H₄X)₂PCH(CH₃)CH(CH₃)P(p-(CH₃)₃NC₆H₄X)₂]₂
Pd[(p-(CH₃)₃NC₆H₄X)₂PC₂H₄P(p-(CH₃)₃NC₆H₄X)₂]₂
et
Pd[(m-SO₃C₆H₄Q)_{c}(C₆H₅)_{2-c}P(CH₂)_{d}P(C₆H₅)_{f}(m-SO₃C₆H₄Q)_{2-f}]₂
[(C₈H₁₂)Pd(P(m-C₆H₄SO₃Q)₃)₂]X₂
[(C₇H₈)Pd(P(CH₂(CH₂CH₂O)ₐCH₃)₃)₂]X₂
[(C₇H₈)Pd(PhP(CH₂CH₂CH₂OCH₃)₂)₂]X₂
[(C₇H₈)Pd(Ph₂PCH₂CH₂N(CH₃)₃X)₂]X₂
[(C₇H₈)Pd((C₆H₅P(m-SO₃C₆H₄Q))₂]X₂
[(C₇H₈)Pd(P(m-SO₃C₆H₄Q)₃)₂]X₂
[(C₈H₁₂)Pd(P(CH₂(CH₂(CH₂CH₂O)ₐCH₃)₃)₂]X₂
[(C₈H₁₂)Pd(PhP(CH₂CH₂CH₂OCH₃)₂)₂]X₂
[(C₇H₈)Pd(p-(CH₃)₃NC₆H₄X)₂PCH(CH₃)CH(CH₃)P(p-(CH₃)₃NC₆H₄X)₂]X₂
[(C₇H₈)Pd(m-SO₃C₆H₄Q)_{c}(C₆H₅)_{2-c}P(CH₂)_{d}P(C₆H₅)_{f}(m-SO₃C₆H₄Q)_{2-f}]X₂
[(C₇H₈)Pd(p-(CH₃)₃NC₆H₄X)₂PC₂H₄P(p-(CH₃)₃NC₆H₄X)₂]X₂ 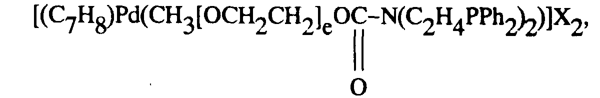
[(C₇H₈)Pd(m-SO₃C₆H₄Q)_{c}(C₆H₅)_{2-c}PCH(CH₃)CH(CH₃)P(C₆H₅)_{f}-(m-SO₃C₆H₄Q)_{2-f}]X₂
[(C₈H₁₂)Pd(p-(CH₃)₃NC₆H₄X)₂PCH(CH₃)CH(CH₃)P(p-(CH₃)₃NC₆H₄X)₂]X₂
[(C₈H₁₂)Pd(p-(CH₃)₃NC₆H₄X)₂PC₂H₄P(p-(CH₃)₃NC₆H₄X)₂]X₂, 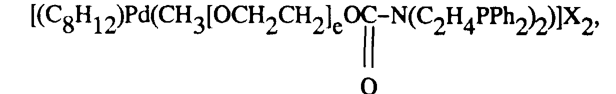
[(C₈H₁₂)Pd(m-SO₃C₆H₄Q)_{c}(C₆H₅)_{2-c}P(CH₂)_{d}P(C₆H₅)_{c}(m-SO₃C₆H₄Q)_{2-c}]X₂
[(C₈H₁₂)Pd(p-(CH₃)₃NC₆H₄X)₂PC₂H₄P(p-(CH₃)₃NC₆H₄X)₂]X₂
[(C₈H₁₂)Pd(m-SO₃C₆H₄Q)_{c}(C₆H₅)_{2-c}PCH(CH₃)CH(CH₃)P(C₆H₅)_{f}-(m-SO₃C₆H₄Q)_{2-f}]X₂
dans lequel Ph est un groupe phényle, P est un atome de phosphore, Pd est un atome de palladium, a est un numéro de 1 à 3, b est un numéro de 1 à 4, c et f sont 1 ou 2, d est un numéro de 2 à 4, e est 12 ou 16, Q est un cation, et X est un anion.
